(19) Europäisches Patentamt
European Patent Office
Office européen des brevets

(11) **EP 4 495 979 A1**

(12) **EUROPEAN PATENT APPLICATION**
published in accordance with Art. 153(4) EPC

(43) Date of publication:
**22.01.2025 Bulletin 2025/04**

(21) Application number: **23770779.9**

(22) Date of filing: **14.03.2023**

(51) International Patent Classification (IPC):
**H01L 21/304** (2006.01)    **H01L 21/306** (2006.01)

(52) Cooperative Patent Classification (CPC):
**H01L 21/304; H01L 21/306**

(86) International application number:
**PCT/JP2023/009827**

(87) International publication number:
**WO 2023/176824 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.03.2022 JP 2022042569**

(71) Applicant: SCREEN Holdings Co., Ltd.
**Kyoto-shi, Kyoto 602-8585 (JP)**

(72) Inventors:
• **SASAKI, Yuta**
  **Kyoto-shi, Kyoto 602-8585 (JP)**
• **HANAWA, Yosuke**
  **Kyoto-shi, Kyoto 602-8585 (JP)**
• **KUNIEDA, Shogo**
  **Kyoto-shi, Kyoto 602-8585 (JP)**

(74) Representative: **Kilian Kilian & Partner mbB**
**Zielstattstraße 23a**
**81379 München (DE)**

(54) **SUBSTRATE TREATMENT METHOD AND TREATMENT LIQUID EVALUATION METHOD**

(57)    The present invention relates to a substrate treating method and a treatment liquid evaluating method. The substrate treating method is to treat a substrate W on which a pattern P is formed. The substrate treating method includes a treatment liquid supply step, a solidified film forming step, and a sublimation step. In the treatment liquid supply step, the treatment liquid L is supplied to the substrate W. The treatment liquid L contains a sublimable substance and a solvent. In the solidified film forming step, the solvent is evaporated from the treatment liquid L on the substrate W. In the solidified film forming step, the solidified film G is formed on the substrate W. The solidified film G contains the sublimable substance. The coefficient K is defined by the interfacial free energies $\gamma LG$, $\gamma GW$, and $yLW$. The coefficient K is equal to or less than the threshold TH.

FIG. 13

## Description

Technical Field

The present invention relates to a substrate treating method and a treatment liquid evaluating method. The substrate is, for example, a semiconductor wafer, a substrate for liquid crystal display, a substrate for organic electroluminescence (EL), a substrate for flat panel display (FPD), a substrate for optical display, a magnetic disk substrate, an optical disk substrate, a magneto-optical disk substrate, a substrate for photomask, a solar cell substrate.

Background Art

Patent Literature 1 discloses a substrate treating method. Specifically, the substrate treating method of Patent Literature 1 includes a treatment liquid supply step, a solidified film forming step, and a sublimation step. In the treatment liquid supply step, a treatment liquid is supplied to a substrate. The treatment liquid contains a sublimable substance and a solvent. In the solidified film forming step, the solvent is evaporated from the treatment liquid on the substrate. In the solidified film forming step, a solidified film is formed on the substrate. The solidified film contains the sublimable substance. In the sublimation step, the solidified film is sublimated. The solidified film changes into a gas without passing through a liquid. The substrate is dried by sublimation of the solidified film.

Citation List

Patent Literature

Patent Literature 1: JP 2021-9988 A

Summary of Invention

Technical Problem

Even in the conventional substrate treating method, there is a case where the substrate cannot be appropriately treated. For example, even in the conventional substrate treating method, a pattern formed on a substrate may collapse.
The present invention has been made in consideration of such circumstances, and a first object thereof is to provide a substrate treating method capable of appropriately treating a substrate. A second object of the present invention is to provide a treatment liquid evaluating method capable of appropriately evaluating a treatment liquid.

Solution to Problem

In order to achieve such an object, the present invention has the following construction. That is, the present invention is a substrate treating method for treating a substrate on which a pattern is formed, the substrate treating method including:

a treatment liquid supply step of supplying a treatment liquid containing a sublimable substance and a solvent to the substrate;
a solidified film forming step of evaporating the solvent from the treatment liquid on the substrate to form a solidified film containing the sublimable substance on the substrate; and
a sublimation step of sublimating the solidified film,
wherein a coefficient K defined by the following equation is equal to or less than a threshold:

$$K = \gamma LG + \gamma GW - \gamma LW$$

where

K represents a coefficient,
$\gamma LG$ represents an interfacial free energy at an interface between the treatment liquid and the solidified film,
$\gamma GW$ represents an interfacial free energy at an interface between the solidified film and the substrate, and
$\gamma LW$ represents an interfacial free energy at an interface between the treatment liquid and the substrate.

The substrate treating method is to treat a substrate on which a pattern is formed. The substrate treating method includes a treatment liquid supply step, a solidified film forming step, and a sublimation step. In the treatment liquid supply step, a treatment liquid is supplied to a substrate. The treatment liquid contains a sublimable substance and a solvent. In the solidified film forming step, the solvent is evaporated from the treatment liquid on the substrate. In the solidified film forming step, a solidified film is formed on the substrate. The solidified film contains the sublimable substance. In the sublimation step, the solidified film is sublimated. The substrate is dried by sublimation of the solidified film.

The coefficient K is defined by the interfacial free energies yLG, yGW, and yLW. The coefficient K is equal to or less than the threshold. The fact that the coefficient K is equal to or less than the threshold is called "first condition", as appropriate. The substrate, the treatment liquid, and the solidified film satisfy the first condition. Accordingly, in the solidified film forming step, the solidified film is suitably formed on the substrate. Specifically, in the solidified film forming step, the solidified film smoothly expands on the substrate. Thus, in the solidified film forming step, the solvent is efficiently removed from the substrate. In the solidified film forming step, the treatment liquid efficiently disappears from the substrate. Therefore, in the sublimation step, the substrate is dried in a state where the pattern formed on the substrate is suitably protected.

As described above, according to the substrate treating method, the substrate is appropriately treated.

In the substrate treating method described above, the threshold is preferably a preset constant. The first condition is simple. Thus, it is easy to manage the substrate treating method so as to satisfy the first condition. Therefore, it is easy to execute the substrate treating method.

In the substrate treating method described above, the threshold is preferably 17 mN/m. In other words, in the substrate treating method described above, the coefficient K is preferably 17 mN/m or less. In the solidified film forming step, the solidified film more smoothly expands on the substrate.

In the substrate treating method described above, the threshold is preferably a variable. The first condition can be set more appropriately. Thus, the substrate can be more appropriately treated.

In the substrate treating method described above, the threshold is preferably a variable that depends on an aspect ratio of the pattern. The first condition is more appropriately set depending on the aspect ratio of the pattern. Thus, regardless of the aspect ratio of the pattern, the substrate is more appropriately treated.

In the substrate treating method described above, the threshold preferably decreases as the aspect ratio increases. As the aspect ratio increases, the pattern collapses more easily. As the threshold decreases, the solidified film more smoothly expands on the substrate. Thus, as the threshold decreases, the pattern is more reliably protected. Therefore, even when the aspect ratio is large, the pattern is suitably protected. Regardless of the aspect ratio, the substrate is appropriately treated.

In the substrate treating method described above, the coefficient K is preferably set depending on the state of the surface of the substrate. Regardless of the state of the surface of the substrate, the coefficient K is appropriately set. Thus, the substrate is appropriately treated regardless of the state of the surface of the substrate.

In the substrate treating method described above, the coefficient K is preferably set based on the state of the surface of the substrate in at least one of the treatment liquid supply step or the solidified film forming step. Regardless of the state of the surface of the substrate in the treatment liquid supply step, the coefficient K is appropriately set. Thus, the substrate is appropriately treated regardless of the state of the surface of the substrate in the treatment liquid supply step. Regardless of the state of the surface of the substrate in the solidified film forming step, the coefficient K is appropriately set. Thus, the substrate is appropriately treated regardless of the state of the surface of the substrate in the solidified film forming step.

In the substrate treating method described above, each of the interfacial free energy yGW and the interfacial free energy yLW is preferably set depending on the state of the surface of the substrate. Accordingly, the interfacial free energy yGW and the interfacial free energy yLW are appropriately set regardless of the state of the surface of the substrate. Thus, when the coefficient K is set, the state of the surface of the substrate is suitably taken into account. Therefore, the coefficient K is appropriately set regardless of the state of the surface of the substrate.

In the substrate treating method described above, each of the interfacial free energy yGW and the interfacial free energy yLW is preferably set based on a state of a surface of the substrate in at least one of the treatment liquid supply step or the solidified film forming step. Regardless of the state of the surface of the substrate in the treatment liquid supply step, the interfacial free energy yGW and the interfacial free energy yLW are appropriately set. Thus, the coefficient K is appropriately set regardless of the state of the surface of the substrate in the treatment liquid supply step. Regardless of the state of the surface of the substrate in the solidified film forming step, the interfacial free energy yGW and the interfacial free energy yLW are appropriately set. Thus, the coefficient K is appropriately set regardless of the state of the surface of the substrate in the solidified film forming step.

In the substrate treating method described above, the surface free energy of the substrate when the surface of the substrate has hydrophobicity is defined as a first surface free energy, the surface free energy of the substrate when the surface of the substrate has hydrophilicity is defined as a second surface free energy. Preferably, the interfacial free energy yGW and the interfacial free energy yLW are each set based on the first surface free energy when the surface of the substrate has the hydrophobicity in the treatment liquid supply step, and the interfacial free energy yGW and the interfacial free energy yLW are each set based on the second surface free energy when the surface of the substrate has the hydrophilicity in the treatment liquid supply step. When the surface of the substrate has hydrophobicity in the treatment liquid supply step, each of the interfacial free energy yGW and the interfacial free energy yLW is appropriately set. When the surface of the substrate has hydrophilicity in the treatment liquid supply step, each of the interfacial free energy yGW and the interfacial free energy yLW is appropriately set. In summary, each of the interfacial free energy yGW and the interfacial free energy yLW is appropriately set regardless of the state of the surface of the substrate in the treatment liquid supply step.

In the substrate treating method described above, the surface free energy of the substrate when the surface of the substrate has hydrophobicity is defined as a first surface free energy, the surface free energy of the substrate when the surface of the substrate has hydrophilicity is defined as a second surface free energy. Preferably, the interfacial free energy yGW and the interfacial free energy yLW are each set based on the first surface free energy when the surface of the substrate has the hydrophobicity in the solidified film forming step, and the interfacial free energy yGW and the interfacial free energy yLW are each set based on the second surface free energy when the surface of the substrate has the hydrophilicity in the solidified film forming step. When the surface of the substrate has hydrophobicity in the solidified film forming step, each of the interfacial free energy yGW and the interfacial free energy yLW is appropriately set. When the surface of the substrate has hydrophilicity in the solidified film forming step, each of the interfacial free energy yGW and the interfacial free energy yLW is appropriately set. In summary, each of the interfacial free energy yGW and the interfacial free energy yLW is appropriately set regardless of the state of the surface of the substrate in the solidified film forming step.

The present invention is a substrate treating method for treating a substrate on which a pattern is formed, the substrate treating method including:

a treatment liquid supply step of supplying a treatment liquid containing a sublimable substance and a solvent to the substrate;

a solidified film forming step of evaporating the solvent from the treatment liquid on the substrate to form a solidified film containing the sublimable substance on the substrate;

a sublimation step of sublimating the solidified film; and

a modification step of modifying a surface of the substrate before the treatment liquid supply step when a coefficient K defined by the following equation is larger than a threshold:

$$K = \gamma LG + \gamma GW - \gamma LW$$

where

K represents a coefficient,

yLG represents an interfacial free energy at an interface between the treatment liquid and the solidified film,

yGW represents an interfacial free energy at an interface between the solidified film and the substrate, and

yLW represents an interfacial free energy at an interface between the treatment liquid and the substrate.

The substrate treating method is to treat a substrate on which a pattern is formed. The substrate treating method includes a treatment liquid supply step, a solidified film forming step, and a sublimation step. In the treatment liquid supply step, a treatment liquid is supplied to a substrate. The treatment liquid contains a sublimable substance and a solvent. In the solidified film forming step, the solvent is evaporated from the treatment liquid on the substrate. In the solidified film forming step, a solidified film is formed on the substrate. The solidified film contains the sublimable substance. In the sublimation step, the solidified film is sublimated. The substrate is dried by sublimation of the solidified film.

Further, the substrate treating method includes a modification step. The modification step is executed when the coefficient K is larger than a threshold. The coefficient K is defined by the interfacial free energies yLG, yGW, and yLW. The modification step is executed before the treatment liquid supply step. The modification step modifies the surface of the substrate. When the coefficient K is larger than the threshold, the surface of the substrate is modified in the modification step, and then the treatment liquid is supplied to the substrate in the treatment liquid supply step. Thus, even when the coefficient K is larger than the threshold, the solidified film is suitably formed on the substrate in the solidified film forming step. Specifically, in the solidified film forming step, the solidified film smoothly expands on the substrate. Therefore, in the solidified film forming step, the solvent is efficiently removed from the substrate. In the solidified film forming step, the treatment liquid efficiently disappears from the substrate. Therefore, in the sublimation step, the substrate is dried in a state where the pattern formed on the substrate is suitably protected.

As described above, according to the substrate treating method, the substrate is appropriately treated.

In the substrate treating method described above, the coefficient K is preferably decreased by the modification step. In the solidified film forming step, the solidified film is more suitably formed on the substrate.

In the substrate treating method described above, the coefficient K is preferably decreased to a value lower than the threshold by the modification step. In the solidified film forming step, the solidified film is more suitably formed on the substrate.

In the substrate treating method described above, in the modification step, the surface of the substrate is preferably modified to be either hydrophilic or hydrophobic. In the modification step, the surface of the substrate can be flexibly modified. Thus, in the solidified film forming step, the solidified film is more suitably formed on the substrate.

In the substrate treating method described above, in the modification step, the surface of the substrate is preferably modified to be hydrophobic. In the modification step, the surface of the substrate can be suitably modified. Thus, in the solidified film forming step, the solidified film is more suitably formed on the substrate.

In the substrate treating method described above, preferably, the modification step is not executed when the coefficient K is equal to or less than the threshold. The time required for the substrate treating method can be effectively shortened. When the coefficient K is equal to or less than the threshold, the solidified film is suitably formed on the substrate in the solidified film forming step even when the modification step is not executed. Thus, even when the coefficient K is equal to or less than the threshold, the substrate is appropriately treated.

The present invention is a substrate treating method for treating a substrate on which a pattern is formed, the substrate treating method including:

a selection step of selecting a treatment liquid containing a sublimable substance and a solvent based on a coefficient K defined by the following equation;
a treatment liquid supply step of supplying the treatment liquid selected by the selection step to the substrate;
a solidified film forming step of evaporating the solvent from the treatment liquid on the substrate to form a solidified film containing the sublimable substance on the substrate; and
a sublimation step of sublimating the solidified film:

$$K = \gamma LG + \gamma GW - \gamma LW$$

where

K represents a coefficient,
$\gamma LG$ represents an interfacial free energy at an interface between the treatment liquid and the solidified film,
$\gamma GW$ represents an interfacial free energy at an interface between the solidified film and the substrate, and
$\gamma LW$ represents an interfacial free energy at an interface between the treatment liquid and the substrate.

The substrate treating method is to treat a substrate on which a pattern is formed. The substrate treating method includes a selection step, a treatment liquid supply step, a solidified film forming step, and a sublimation step. In the selection step, a treatment liquid is selected. The treatment liquid contains a sublimable substance and a solvent. In the treatment liquid supply step, the treatment liquid selected in the selection step is supplied to the substrate. In the solidified film forming step, the solvent is evaporated from the treatment liquid on the substrate. In the solidified film forming step, a solidified film is formed on the substrate. The solidified film contains the sublimable substance. In the sublimation step, the solidified film is sublimated. The substrate is dried by sublimation of the solidified film.

Here, in the selection step, the treatment liquid is selected based on the coefficient K. The coefficient K is defined by the interfacial free energies yLG, yGW, and yLW. Accordingly, in the selection step, the treatment liquid is appropriately selected. Thus, in the solidified film forming step, the solidified film is suitably formed on the substrate. Specifically, in the solidified film forming step, the solidified film smoothly expands on the substrate. Therefore, in the solidified film forming step, the solvent is efficiently removed from the substrate. In the solidified film forming step, the treatment liquid efficiently disappears from the substrate. Therefore, in the sublimation step, the substrate is dried in a state where the pattern formed on the substrate is suitably protected.

As described above, according to the substrate treating method, the substrate is appropriately treated.

In the substrate treating method described above, in the selection step, the treatment liquid that causes the coefficient K to be equal to or less than the threshold is preferably selected. In other words, in the substrate treating method described above, in the selection step, it is preferable to select the treatment liquid satisfying that the coefficient K is equal to or less than the threshold. In the selection step, the treatment liquid is more appropriately selected.

In the substrate treating method described above, the coefficient K is preferably set depending on the state of the surface of the substrate. Regardless of the state of the surface of the substrate, the coefficient K is appropriately set. Thus, in the selection step, the treatment liquid is selected depending on the state of the surface of the substrate. In the selection step, the treatment liquid is appropriately selected regardless of the state of the surface of the substrate. Therefore, in the treatment liquid supply step, the solidified film forming step, and the sublimation step, the substrate is appropriately treated regardless of the state of the surface of the substrate.

In the substrate treating method described above, the coefficient K is preferably set based on the state of the surface of the substrate in at least one of the treatment liquid supply step or the solidified film forming step. The substrate is appropriately treated regardless of the state of the surface of the substrate in the treatment liquid supply step. The substrate is appropriately treated regardless of the state of the surface of the substrate in the solidified film forming step.

Further, the present invention is a treatment liquid evaluating method that evaluates a treatment liquid for treating a substrate on which a pattern is formed,

in which the treatment liquid contains a sublimable substance and a solvent, and the solvent evaporates from the treatment liquid, and thus the treatment liquid becomes a solidified film containing the sublimable substance, and the treatment liquid evaluating method includes: an acquisition step of acquiring a coefficient K defined by the following equation; and
an evaluation step of evaluating the treatment liquid based on the coefficient K:

$$K = \gamma LG + \gamma GW - \gamma LW$$

where

K represents a coefficient,
$\gamma LG$ represents an interfacial free energy at an interface between the treatment liquid and the solidified film,
$\gamma GW$ represents an interfacial free energy at an interface between the solidified film and the substrate, and
$\gamma LW$ represents an interfacial free energy at an interface between the treatment liquid and the substrate.

The treatment liquid evaluating method is to evaluate a treatment liquid. The treatment liquid is to treat a substrate on which a pattern is formed. The treatment liquid contains a sublimable substance and a solvent. If the solvent evaporates from the treatment liquid, the treatment liquid becomes a solidified film. The solidified film contains the sublimable substance. The treatment liquid evaluating method includes an acquisition step and an evaluation step. The acquisition step acquires the coefficient K. The coefficient K is defined by the interfacial free energies $\gamma LG$, $\gamma GW$, and $\gamma LW$. In the evaluation step, the treatment liquid is evaluated based on the coefficient K. Thus, the treatment liquid is suitably evaluated from the viewpoint of the quality of the treatment of the substrate.

As described above, according to the treatment liquid evaluating method, the treatment liquid is appropriately evaluated. The treatment liquid evaluating method is useful in selecting a treatment liquid.

In the treatment liquid evaluating method described above, in the acquisition step, the coefficient K is preferably acquired depending on the state of the surface of the substrate. In the acquisition step, the coefficient K is appropriately acquired regardless of the state of the surface of the substrate. Thus, in the evaluation step, the treatment liquid is evaluated in consideration of the state of the surface of the substrate. In the evaluation step, the treatment liquid is appropriately evaluated regardless of the state of the surface of the substrate.

In the treatment liquid evaluating method described above, preferably, the coefficient K includes a first coefficient when a surface of the substrate has hydrophobicity, and a second coefficient when a surface of the substrate has hydrophilicity, and in the acquisition step, the first coefficient and the second coefficient are acquired, and in the evaluation step, the treatment liquid is evaluated based on the first coefficient and the second coefficient. In the acquisition step, the first coefficient and the second coefficient are acquired. The first coefficient corresponds to the coefficient K when the surface of the substrate has hydrophobicity. The second coefficient corresponds to the coefficient K when the surface of the substrate has hydrophilicity. In the evaluation step, the treatment liquid is evaluated based on the first coefficient. In the evaluation step, the treatment liquid is evaluated based on the second coefficient. Thus, in the evaluation step, the treatment liquid is evaluated in consideration of the state of the surface of the substrate. In the evaluation step, the treatment liquid is appropriately evaluated regardless of the state of the surface of the substrate.

In the treatment liquid evaluating method described above, the acquisition step preferably includes a first step of preparing a first substrate having a surface that is hydrophobic and a second substrate having a surface that is hydrophilic, a second step of acquiring the coefficient K based on the first substrate, and a third step of acquiring the coefficient K based on the second substrate. In the acquisition step, the coefficient K is appropriately acquired regardless of the state of the surface of the substrate.

In the treatment liquid evaluating method described above, in the acquisition step, the coefficient K is preferably acquired based on the state of the surface of the substrate and the composition of the treatment liquid. In the acquisition step, the coefficient K is appropriately acquired.

In the treatment liquid evaluating method described above, in the acquisition step, the coefficient K is preferably selected from preset information regarding the coefficient K. In the acquisition step, the coefficient K is easily acquired.

In the treatment liquid evaluating method described above, in the evaluation step, preferably, the treatment liquid that causes the coefficient K to be equal to or less than a threshold is classified into a first class, and the treatment liquid that causes the coefficient K to be larger than the threshold is classified into a second class. In other words, in the treatment liquid evaluating method described above, preferably, a first condition is that the coefficient K is equal to or less than a threshold, and in the evaluation step, the treatment liquid satisfying the first condition is classified into a first class, and the treatment liquid not satisfying the first condition is classified into a second class. In the evaluation step, the treatment liquid is classified into one of the first class and the second class based on the coefficient K. Thus, in the evaluation step, the treatment liquid is clearly evaluated.

Advantageous Effects of Invention

According to the substrate treating method of the present invention, the substrate is appropriately treated. According to the treatment liquid evaluating method of the present invention, the treatment liquid is appropriately evaluated.

Brief Description of Drawings

FIG. 1 is a view schematically illustrating a part of a substrate.
FIG. 2 is a plan view illustrating an interior of a substrate treating apparatus according to a first embodiment.
FIG. 3 is a control block diagram of the substrate treating apparatus.
FIG. 4 is a diagram illustrating a construction of a treating unit.
FIG. 5 is a flowchart showing procedures of a substrate treating method according to the first embodiment.
FIG. 6 is a view schematically illustrating the substrate in a treatment liquid supply step.
FIG. 7 is a view schematically illustrating the substrate in a solidified film forming step.
FIG. 8 is an enlarged view schematically illustrating the substrate in the solidified film forming step.
FIG. 9 is a view schematically illustrating the substrate in a sublimation step.
FIG. 10 is a view schematically illustrating the substrate in the sublimation step.
FIG. 11 is a flowchart showing procedures of a substrate treating method according to a first embodiment.
FIG. 12 is a table showing coefficients and average collapse rates in Examples.
FIG. 13 is a graph showing a relationship between coefficients and average collapse rates in Examples.
FIG. 14 is a flowchart showing procedures of a substrate treating method according to the second embodiment.
FIG. 15 is a diagram illustrating a construction of a treating unit according to a third embodiment.
FIG. 16 is a flowchart showing procedures of the substrate treating method according to the third embodiment.
FIG. 17 is a flowchart showing procedures of a treatment liquid evaluating method according to a fourth embodiment.

Description of Embodiments

Hereinafter, a substrate treating method and a treatment liquid evaluating method of the present invention will be described with reference to the drawings.

<1. First Embodiment>

<1-1. Substrate>

A substrate W is, for example, a semiconductor wafer, a substrate for liquid crystal display, a substrate for organic electroluminescence (EL), a substrate for flat panel display (FPD), a substrate for optical display, a magnetic disk substrate, an optical disk substrate, a magneto-optical disk substrate, a substrate for photomask, a solar cell substrate. The substrate W has a thin and flat plate shape. The substrate W has a substantially circular shape in plan view.

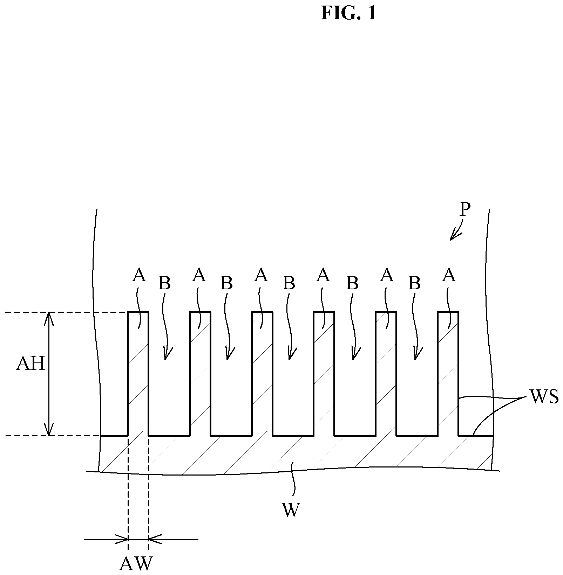

FIG. 1 is a view schematically illustrating a part of the substrate W. The substrate W has a pattern P. The pattern P is formed on a surface WS of the substrate W. The pattern P has, for example, an uneven shape.

The pattern P has, for example, a plurality of projections A. The projections A are part of the substrate W. The projections A are a structure. The projections A are each formed with, for example, at least one of a monocrystalline silicon film, a silicon oxide ($SiO_2$) film, a silicon nitride (SiN) film, or a polysilicon film. The projections A project upward, for example. The plurality of projections A is arranged laterally, for example. The plurality of projections A are separated from each other.

The projection A defines the recess B. The recess B is a space. The recess B is located between two projections A adjacent to each other. The recess B is opened upward, for example.

<1-2. Outline of Substrate Treating Apparatus>

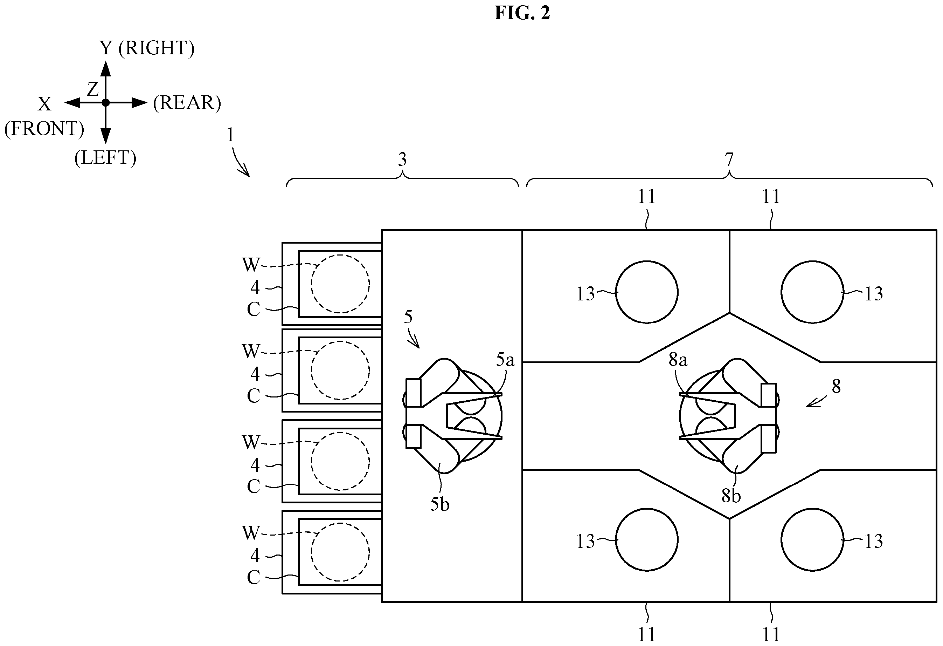

FIG. 2 is a plan view illustrating an interior of a substrate treating apparatus 1 according to a first embodiment. A substrate treating apparatus 1 performs treatment on the substrate W. The treatment in the substrate treating apparatus 1 includes a dry treatment.

The substrate treating apparatus 1 includes an indexer 3 and a treating block 7. The treating block 7 is connected to the indexer 3. The indexer 3 supplies the substrate W to the treating block 7. The treating block 7 performs treatment on the substrate W. The indexer 3 collects the substrate W from the treating block 7.

In this specification, the direction in which the indexer 3 and the treating block 7 are arranged is called "front-rear direction X" for convenience. The front-rear direction X is horizontal. One direction of the front-rear direction X from the treating block 7 to the indexer 3 is called "forward direction". The direction opposite to the forward direction is called "rearward direction". The horizontal direction orthogonal to the front-rear direction X is called "transverse direction Y". One direction in the "transverse direction Y" is called "rightward direction", as appropriate. The direction opposite to the rightward direction is called "leftward direction". The perpendicular direction relative to the horizontal direction is called "vertical direction Z". For reference, the drawings show front, rear, right, left, up, and down, as appropriate.

The indexer 3 includes a plurality of (e.g. four) carrier platforms 4. The carrier platforms 4 each include one carrier C placed thereon. The carrier C accommodates a plurality of substrates W. The carrier C is, for example, a front opening unified pod (FOUP), a standard mechanical interface (SMIF), or an open cassette (OC).

The indexer 3 includes a transport mechanism 5. The transport mechanism 5 is arranged rearward of the carrier platforms 4. The transport mechanism 5 transports the substrate W. The transport mechanism 5 is configured to access the carriers C placed on the carrier platforms 4.

The transport mechanism 5 includes a hand 5a and a hand driving unit 5b. The hand 5a supports the substrate W. The hand driving unit 5b is coupled to the hand 5a. The hand driving unit 5b moves the hand 5a. The hand driving unit 5b moves the hand 5a in the front-rear direction X, transverse direction Y, and vertical direction Z, for example. The hand driving unit 5b rotates the hand 5a in a horizontal plane, for example.

The treating block 7 includes a transport mechanism 8. The transport mechanism 8 transports the substrate W. The transport mechanism 8 is configured to receive the substrate W from the transport mechanism 5 and to deliver the substrate W to the transport mechanism 5.

The transport mechanism 8 includes a hand 8a and a hand driving unit 8b. The hand 8a supports the substrate W. The hand driving unit 8b is coupled to the hand 8a. The hand driving unit 8b moves the hand 8a. The hand driving unit 8b moves the hand 8a in the front-rear direction X, transverse direction Y, and vertical direction Z, for example. The hand driving unit 8b rotates the hand 8a in a horizontal plane, for example.

The treating block 7 includes a plurality of treating units 11. The treating units 11 are each arranged laterally of the transport mechanism 8. The treating units 11 each perform a treatment on the substrate W.

Each of the treating units 11 includes a substrate holder 13. The substrate holder 13 holds the substrate W.

The transport mechanism 8 is configured to access each of the treating units 11. The transport mechanism 8 is configured to deliver the substrate W to the substrate holder 13 and to take the substrate W from the substrate holder 13.

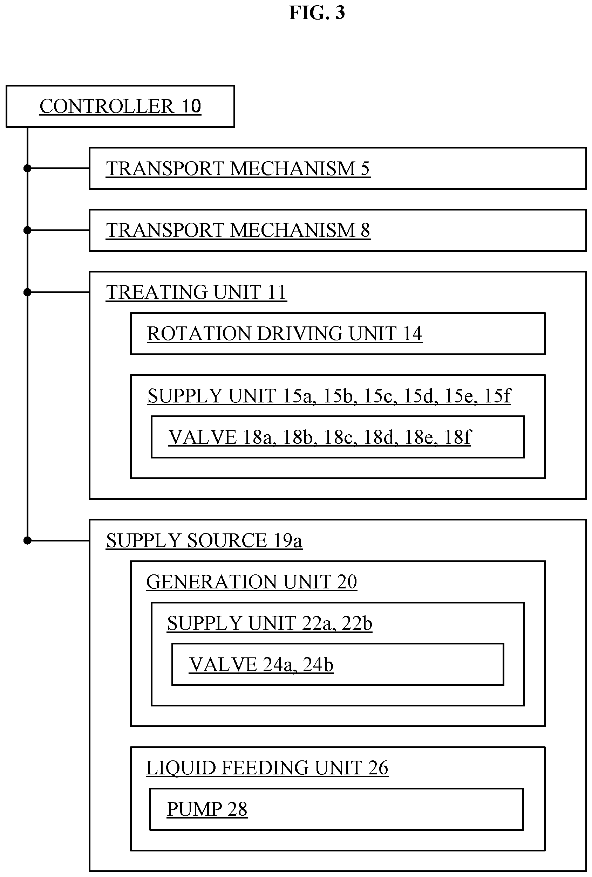

FIG. 3 is a control block diagram of the substrate treating apparatus 1. The substrate treating apparatus 1 includes a controller 10. The controller 10 is communicably connected to the transport mechanisms 5 and 8 and the treating unit 11. The controller 10 controls the transport mechanisms 5 and 8 and the treating units 11.

The controller 10 is implemented by a central processing unit (CPU) that performs various processes, a random-access memory (RAM) as a workspace of arithmetic processing, a storage medium such as a fixed disk. The controller 10 has various types of information stored in the storage medium in advance. The information included in the controller 10 includes transportation condition information and processing condition information, for example. The transportation condition information defines a condition related to the transportation of the substrate W by the transport mechanisms 5 and 8. The processing condition information defines a condition related to the processing of the substrate W in the treating unit 11. The processing condition information is also called as processing recipes.

An operation example of the substrate treating apparatus 1 will be simply described.

The indexer 3 supplies the substrate W to the treating block 7. Specifically, the transport mechanism 5 delivers the substrate W from each of the carriers C to the transport mechanism 8 of the treating block 7.

The transport mechanism 8 distributes the substrate W to each of the treating units 11. Specifically, the transport mechanism 8 transports the substrate W from the transport mechanism 5 to the substrate holders 13 of each of the treating units 11.

The treating unit 11 performs treatment on the substrate W held by the substrate holder 13. The treating unit 11 performs a dry treatment, for example, on the substrate W.

After the treating unit 11 performs treatment on the substrate W, the transport mechanism 8 collects the substrate W from each of the treating units 11. Specifically, the transport mechanism 8 receives the substrate W from each of the substrate holders 13. Then, the transport mechanism 8 delivers the substrate W to the transport mechanism 5.

The indexer 3 collects the substrate W from the treating block 7. Specifically, the transport mechanism 5 transports the substrate W from the transport mechanism 8 to the carrier C.

<1-3. Construction of Treating Unit 11>

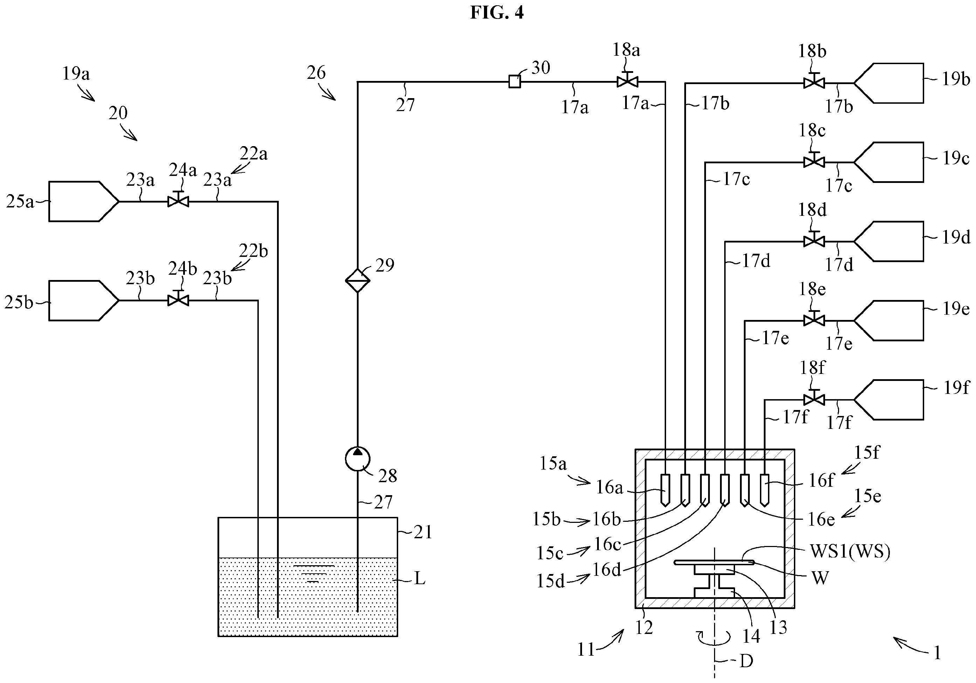

FIG. 4 is a diagram illustrating a construction of the treating unit 11. The treating units 11 each have the same structure. The treating unit 11 is classified as a single-wafer processing unit. That is, the treating units 11 each perform a treatment on only one substrate W at one time.

The treating unit 11 includes a casing 12. The casing 12 has a substantial box shape. The substrate W is treated in the interior of the casing 12.

The interior of the casing 12 is kept at normal temperature, for example. Accordingly, the substrate W is treated under an environment of normal temperature, for example. Here, the normal temperature includes room temperature. The normal temperature is, for example, a temperature within a range of 5°C to 35°C. The normal temperature is, for example, a temperature within a range of 10°C to 30°C. The normal temperature is, for example, a temperature within a range of 15°C to 25°C.

The interior of the casing 12 is kept at normal pressure, for example. Accordingly, the substrate W is treated under an environment of normal pressure, for example. Here, the normal pressure includes standard atmospheric pressure (1 atm, 101325 Pa). The normal pressure falls within a pressure range of 0.7 to 1.3 atm, for example. In this specification, the value of pressure is indicated as absolute pressure relative to absolute vacuum.

The substrate holder 13 described above is installed the interior of the casing 12. The substrate holder 13 holds one substrate W. The substrate holder 13 holds the substrate W in a substantially horizontal posture.

The substrate holder 13 is located below the substrate W held by the substrate holder 13. The substrate holder 13 comes into contact with at least one of a lower surface of the substrate W or a peripheral edge of the substrate W. The lower surface of the substrate W is also called a back side of the substrate W. The substrate holder 13 is not in contact with an upper surface WS1 of the substrate W. Here, the upper surface WS1 is directed upward. The upper surface WS1 is a part of the surface WS.

The treating unit 11 includes a rotation driving unit 14. At least a part of the rotation driving unit 14 is installed in the interior of the casing 12. The rotation driving unit 14 is connected to the substrate holder 13. The rotation driving unit 14 rotates the substrate holder 13. The substrate W held by the substrate holder 13 rotates integrally with the substrate holder 13. The substrate W held by the substrate holder 13 rotates about a rotation axis D, for example. The rotation axis D passes through the center of the substrate W, for example. The rotation axis D extends in the vertical direction Z, for example.

The treating unit 11 includes supply units 15a, 15b, 15c, 15d, 15e, and 15f. Each of the supply units 15a to 15f supplies a liquid or gas to the substrate W held by the substrate holder 13. Specifically, each of the supply units 15a to 15f supplies a liquid or gas to the upper surface WS1 of the substrate W held by the substrate holder 13.

The supply unit 15a supplies a treatment liquid L. The supply unit 15b supplies a chemical liquid. The supply unit 15c supplies a cleaning liquid. The supply unit 15d supplies a rinse liquid. The supply unit 15e supplies a replacement liquid. The supply unit 15f supplies a dry gas.

The treatment liquid L supplied by the supply unit 15a contains a sublimable substance and a solvent.

The sublimable substance has sublimability. "Sublimability" means a property that a single substance, a compound, or a mixture changes its phase from a solid phase to a gas phase or from a gas phase to a solid phase without passing through a liquid phase. The sublimable substance preferably has a vapor pressure of 0.1 Pa or more at normal temperature. For example, the sublimable substance contains at least one of cyclohexanone oxime, camphor, pinacolin oxime, acetophenone oxime, cyclopentanone oxime, 4-tert butylphenol, 4-nitrotoluene, or ε-caprolactam. For example, the sublimable substance is composed of only cyclohexanone oxime. For example, the sublimable substance is composed of only camphor.

The solvent is a liquid at normal temperature. The solvent dissolves the sublimable substance. Accordingly, the sublimable substance in the treatment liquid L is dissolved in the solvent. That is, the treatment liquid L contains the solvent and the sublimable substance dissolved in the solvent. The sublimable substance corresponds to a solute of the treatment liquid L. The solvent preferably has a relatively high vapor pressure at normal temperature. For example, the vapor pressure of the solvent at normal temperature is preferably higher than the vapor pressure of the sublimable substance at normal temperature. The solvent is, for example, an organic solvent. The solvent is, for example, an alcohol. The solvent contains, for example, at least one of isopropyl alcohol (IPA), methanol, ethanol, 1-propanol, isobutanol, propylene glycol monomethyl ether acetate (PGMEA), 1-ethoxy-2-propanol (PGEE), acetone, or 1-butanol. For example, the solvent is composed of only isopropyl alcohol. For example, the solvent is composed of only methanol.

The treatment liquid L is composed of only a sublimable substance and a solvent, for example. For example, the treatment liquid L is composed of only cyclohexanone oxime and isopropyl alcohol. For example, the treatment liquid L is composed of only camphor and methanol.

The treatment liquid L is used to dry the substrate W. The treatment liquid L has a function of assisting drying of the substrate W. Accordingly, the treatment liquid L may be called "dry assistant liquid".

The chemical liquid supplied by the supply unit 15b is, for example, an etching liquid. The etching liquid contains, for example, at least one of hydrofluoric acid (HF) or buffered hydrofluoric acid (BHF).

The cleaning liquid supplied by the supply unit 15c is, for example, SC1. SC1 is a mixed liquid of ammonia, hydrogen peroxide, and deionized water. SC1 is also called "APM" or "ammonia hydrogen peroxide mixture".

The rinse liquid supplied by the supply unit 15d is, for example, deionized water (DIW).

The replacement liquid supplied by the supply unit 15e is, for example, an organic solvent. The replacement liquid is, for example, isopropyl alcohol (IPA).

The dry gas supplied by the supply unit 15f preferably has a dew point lower than normal temperature. Preferably, the dry gas does not condense at normal temperature. The dry gas contains at least one of air or inert gas. The air is, for example, compressed air. The inert gas is, for example, nitrogen gas.

The supply unit 15a includes a nozzle 16a. Likewise, the supply units 15b to 15f include nozzles 16b to 16f, respectively. The nozzle 16a dispenses the treatment liquid L. The nozzle 16b dispenses the chemical liquid. The nozzle 16c dispenses the cleaning liquid. The nozzle 16d dispenses the rinse liquid. The nozzle 16e dispenses the replacement liquid. The nozzle 16f dispenses the dry gas.

Each of the nozzles 16a to 16f is installed in the interior of the casing 12. Each of the nozzles 16a to 16f is movable to a treatment position and a standby position. The treatment position is, for example, a position above the substrate W held by the substrate holder 13. The standby position is, for example, a position deviated from above the substrate W held by the substrate holder 13.

The treatment liquid L is used in the interior of the casing 12. As described above, the interior of the casing 12 is kept at normal temperature, for example. Accordingly, the treatment liquid L is used, for example, under an environment of normal temperature. The interior of the casing 12 is kept at normal pressure, for example. Accordingly, the treatment liquid L is used, for example, under an environment of normal pressure. The chemical liquid, the cleaning liquid, the rinse liquid, the replacement liquid, and the dry gas are also used, for example, under an environment of normal temperature and normal pressure.

The supply unit 15a includes a pipe 17a and a valve 18a. The pipe 17a is connected to the nozzle 16a. The valve 18a is provided on the pipe 17a. When the valve 18a opens, the nozzle 16a dispenses the treatment liquid L. When the valve 18a closes, the nozzle 16a does not dispense the treatment liquid L. Likewise, the supply units 15b to 15f includes pipes 17b to 17f and valves 18b to 18f, respectively. The pipes 17b to 17f are connected to the nozzles 16b to 16f, respectively. The valves 18b to 18f are provided on the pipes 17b to 17f, respectively. The valves 18b to 18f each control dispensing of the chemical liquid, the cleaning liquid, the rinse liquid, the replacement liquid, and the dry gas.

At least a part of the pipe 17a may be provided externally of the casing 12. The pipes 17b to 17f may also be arranged in a similar manner to the pipe 17a. The valve 18a may be provided externally of the casing 12. The valves 18b to 18f may also be arranged in a similar manner to the valve 18a.

The supply unit 15a is connected to a supply source 19a. The supply source 19a is connected to, for example, the pipe 17a. The supply source 19a feeds the treatment liquid L to the supply unit 15a. Likewise, the supply units 15b to 15f are connected to supply sources 19b to 19f, respectively. The supply sources 19b to 19f are connected to, for example, the pipes 17b to 17f, respectively. The supply source 19b feeds the chemical liquid to the supply unit 15b. The supply source 19c feeds the cleaning liquid to the supply unit 15c. The supply source 19d feeds the rinse liquid to the supply unit 15d. The supply source 19e feeds the replacement liquid to the supply unit 15e. The supply source 19f feeds the dry gas to the supply unit 15f.

The supply source 19a is provided externally of the casing 12. Likewise, the supply sources 19b to 19f each are provided externally of the casing 12.

The supply source 19a may supply the treatment liquid L to the plurality of treating units 11. Alternatively, the supply source 19a may supply the treatment liquid L to only one treating unit 11. The same applies to the supply sources 19b to 19f.

The supply source 19a may be an element of the substrate treating apparatus 1. For example, the supply source 19a may be installed in the interior of the substrate treating apparatus 1. Alternatively, the supply source 19a need not be an element of the substrate treating apparatus 1. For example, the supply source 19a may be installed externally of the substrate treating apparatus 1. Likewise, each of the supply sources 19b to 19f may be an element of the substrate treating apparatus 1. Alternatively, each of the supply sources 19b to 19f need not be an element of the substrate treating apparatus 1.

The treating unit 11 may further include a cup, not illustrated. The cup is installed in the interior of the casing 12. The cup is arranged laterally of the substrate holder 13. The cup surrounds an exterior of the substrate holder 13. The cup receives the liquid scattered from the substrate W held by the substrate holder 13.

Reference is made to FIG. 3. The controller 10 controls the rotation driving unit 14. The controller 10 controls the supply units 15a to 15f. The controller 10 controls the valves 18a to 18f.

The controller 10 is communicably connected to the supply source 19a, for example. The controller 10 controls the supply source 19a, for example.

<1-4. Construction of Supply Source 19a>

Reference is made to FIG. 4. The supply source 19a includes a generation unit 20. The generation unit 20 generates the treatment liquid L. The generation unit 20 stores the treatment liquid L.

The generation unit 20 includes a tank 21 and supply units 22a and 22b. The supply unit 22a supplies the sublimable substance to the tank 21. The supply unit 22b supplies the solvent to the tank 21. The sublimable substance and the solvent are mixed in the tank 21. The treatment liquid L is generated in the tank 21. The treatment liquid L is stored in the tank 21.

Specifically, the supply unit 22a includes a pipe 23a and a valve 24a. The pipe 23a is connected to the tank 21. The valve 24a is provided on the pipe 23a. When the valve 24a opens, the supply unit 22a supplies the sublimable substance to the tank 21. When the valve 24a closes, the supply unit 22a does not supply the sublimable substance to the tank 21. Likewise, the supply unit 22b includes a pipe 23b and a valve 24b. The pipe 23b is connected to the tank 21. The valve 24b is provided on the pipe 23b. The valve 24b controls the supply of the solvent to the tank 21.

The supply unit 22a is connected to a supply source 25a. The supply source 25a is connected to, for example, the pipe 23a. The supply source 25a feeds the sublimable substance to the supply unit 22a. Likewise, the supply unit 22b is connected to a supply source 25b. The supply source 25b is connected to, for example, the pipe 23b. The supply source 25b feeds the solvent to the supply unit 22b.

The supply source 19a includes a liquid feeding unit 26. The liquid feeding unit 26 feeds the treatment liquid L from the generation unit 20 to the supply unit 15a. The liquid feeding unit 26 includes, for example, a pipe 27, a pump 28, a filter 29, and a joint 30. The pipe 27 is connected to the tank 21. The pump 28 is provided on the pipe 27. The filter 29 is provided on the pipe 27. The joint 30 is connected to the pipe 27. The joint 30 is further connected to the pipe 17a.

The pump 28 feeds the treatment liquid L from the tank 21 to the pipe 17a through the pipe 27. The filter 29 filters the treatment liquid L flowing through the pipe 27. The filter 29 removes foreign substances from the treatment liquid L.

Reference is made to FIG. 3. The controller 10 controls the generation unit 20. The controller 10 controls the supply units 22a and 22b. The controller 10 controls the valves 24a and 24b. The controller 10 controls the liquid feeding unit 26. The controller 10 controls the pump 28.

<1-5. First Operation Example of Supply Source 19a and Treating Unit 11>

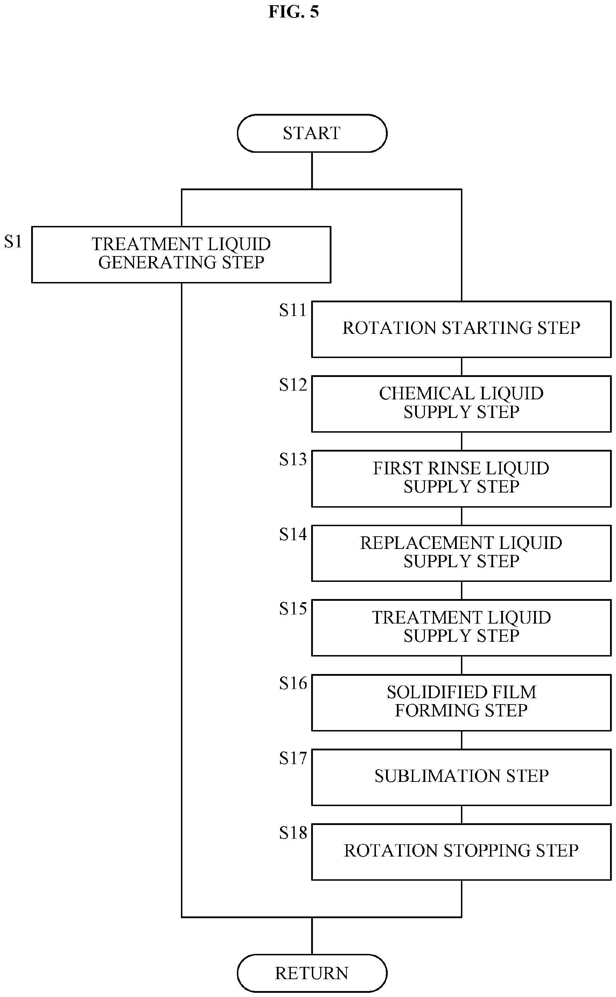

Reference is made to FIGS. 4 and 5. FIG. 5 is a flowchart showing procedures of a substrate treating method according to the first embodiment. FIG. 5 shows procedures of a first operation example. In the first operation example, the substrate treating method includes Step S1 and Steps S11 to S18. Step S1 is executed by the supply source 19a. Steps S11 to S18 are substantially executed by the treating unit 11. Step S1 is executed in parallel with Steps S11 to S18. The supply source 19a and the treating unit 11 operate in accordance with control of the controller 10.

Step S1: Treatment liquid generating step

The treatment liquid L is generated.

The generation unit 20 generates the treatment liquid L in the tank 21. The generation unit 20 stores the treatment liquid L in the tank 21.

Step S11: Rotation starting step

The substrate W starts to rotate.

The substrate holder 13 holds the substrate W. The substrate W is held in a substantially horizontal posture. The rotation driving unit 14 starts to rotate the substrate holder 13. The substrate W rotates integrally with the substrate holder 13. In Steps S12 to S17, the substrate W continues to rotate, for example.

Step S12: Chemical liquid supply step

The chemical liquid is supplied to the substrate W.

The supply unit 15b supplies the chemical liquid to the substrate W held by the substrate holder 13. The chemical liquid is supplied to the upper surface WS1 of the substrate W. For example, the chemical liquid etches the substrate W. For example, the chemical liquid removes a natural oxide film from the substrate W. Then, the supply unit 15b stops supplying the chemical liquid to the substrate W.

When the chemical liquid is hydrofluoric acid, the chemical liquid terminates the upper surface WS1 of the substrate W with hydrogen. For example, hydrogen is bonded to an atom (e.g. a silicon atom) located on the upper surface WS1 of the substrate W. Accordingly, the upper surface WS1 of the substrate W is modified to be hydrophobic.

Step S13: First rinse liquid supply step

The rinse liquid is supplied to the substrate W.

The supply unit 15c supplies the rinse liquid to the substrate W held by the substrate holder 13. The rinse liquid is supplied to the upper surface WS1 of the substrate W. The rinse liquid removes the chemical liquid from the substrate W. The chemical liquid on the substrate W is replaced with the rinse liquid. Then, the supply unit 15c stops supplying the rinse liquid to the substrate W.

Even after the rinse liquid supply step, the substrate W is still terminated with hydrogen. Accordingly, even after the first rinse liquid supply step, the upper surface WS1 of the substrate W has hydrophobicity.

Step S14: Replacement liquid supply step

The replacement liquid is supplied to the substrate W.

The supply unit 15d supplies the replacement liquid to the substrate W held by the substrate holder 13. The replacement liquid is supplied to the upper surface WS1 of the substrate W. The replacement liquid removes the rinse liquid from the substrate W. The rinse liquid on the substrate W is replaced with the replacement liquid. Then, the supply unit 15d stops supplying the replacement liquid to the substrate W.

Even after the replacement liquid supply step, the substrate W is still terminated with hydrogen. Accordingly, even after the replacement liquid supply step, the upper surface WS1 of the substrate W has hydrophobicity.

Step S15: Treatment liquid supply step

In the treatment liquid supply step, the upper surface WS1 of the substrate W has hydrophobicity. The treatment liquid L is supplied to the substrate W.

The liquid feeding unit 26 supplies the treatment liquid L from the generation unit 20 to the supply unit 15a. The supply unit 15a supplies the treatment liquid L to the substrate W held by the substrate holder 13. The treatment liquid L is supplied to the upper surface WS1 of the substrate W. The treatment liquid L removes the replacement liquid from the substrate W. The replacement liquid on the substrate W is replaced with the treatment liquid L. Then, the liquid feeding unit 26 stops supplying the treatment liquid L to the supply unit 15a. The supply unit 15a stops supplying the treatment liquid L to the substrate W.

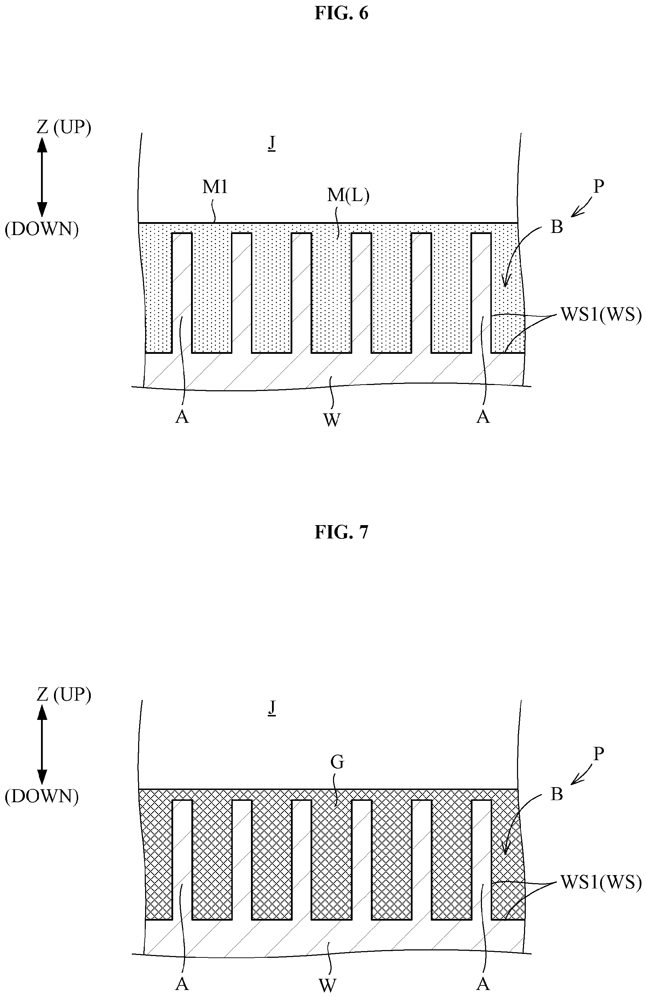

FIG. 6 is a view schematically illustrating the substrate W in the treatment liquid supply step. When the substrate W is held by the substrate holder 13, the pattern P is located on the upper surface WS1 of the substrate W. When the substrate W is held by the substrate holder 13, the pattern P is directed upward.

The treatment liquid L on the substrate W forms a liquid film M. The liquid film M is located on the substrate W. The liquid film M is located on the upper surface WS1. The liquid film M covers the upper surface WS1.

The pattern P comes into contact with the treatment liquid L. The projection A comes into contact with the treatment liquid L.

The pattern P is entirely immersed in the liquid film M. The entire projection A is immersed in the liquid film M. The liquid film M has an upper surface M1. The upper surface M1 is located at a position higher than the pattern P. The upper surface M1 is located at a position higher than the projection A. The recess B is filled with the liquid film M. The entire recess B is filled with only the liquid film M.

The replacement liquid has already been removed from the substrate W by the treatment liquid L. Accordingly, the replacement liquid is not present on the substrate W. The replacement liquid does not remain in the recesses B.

The liquid film M comes into contact with a gas J. The upper surface M1 comes into contact with the gas J. The pattern P is not in contact with the gas J. The pattern P is not exposed to the gas J. The projection A is not in contact with the gas J. The projection A is not exposed to the gas J. The gas J corresponds to the atmosphere in the casing 12.

In the treatment liquid supply step, the height position of the upper surface M1 may be further adjusted. For example, the height position of the upper surface M1 may be adjusted while the supply unit 15a supplies the treatment liquid L to the substrate W. For example, the height position of the upper surface M1 may be adjusted after the supply unit 15a stops supplying the treatment liquid L. For example, the height position of the upper surface M1 may be adjusted by adjusting a rotation speed of the substrate W. For example, the height position of the upper surface M1 may be adjusted by adjusting a period of rotation of the substrate W. Adjusting the height position of the upper surface M1 corresponds to adjusting the thickness of the liquid film M.

Even after the treatment liquid supply step, the substrate W is still terminated with hydrogen. Accordingly, even after the treatment liquid supply step, the upper surface WS1 of the substrate W has hydrophobicity.

Step S16: Solidified film forming step

In the solidified film forming step, the upper surface WS1 of the substrate W has hydrophobicity. The solvent evaporates from the treatment liquid L on the substrate W. A solidified film is formed on the substrate W. The solidified film contains the sublimable substance.

FIG. 7 is a view schematically illustrating the substrate W in the solidified film forming step. As described above, the solvent has a relatively high vapor pressure. Accordingly, the solvent smoothly evaporates from the treatment liquid L on the substrate W. The solvent smoothly changes from a liquid to gas.

As the solvent evaporates from the treatment liquid L, the amount of the solvent in the treatment liquid L decreases. As the amount of the solvent in the treatment liquid L decreases, the concentration of the sublimable substance in the treatment liquid L increases. Eventually, the sublimable substance in the treatment liquid L starts to precipitate. The sublimable substance starts to precipitate from the treatment liquid L. The sublimable substance changes from the solute of the treatment liquid L to a solid. The solid sublimable substance forms a solidified film G. That is, the solidified film G corresponds to the solid sublimable substance. The solidified film G contains the sublimable substance. The solidified film G does not contain a solvent. The solidified film G is a solid. As described above, the solvent evaporates from the treatment liquid L, and the sublimable substance precipitates from the treatment liquid L, and thus the treatment liquid L changes to the solidified film G. The treatment liquid L changes to the solidified film G, and thus the treatment liquid L decreases. The treatment liquid L changes to the solidified film G, and thus the liquid film M becomes thinner.

The solidified film G is formed on the substrate W.

The solvent further evaporates from the treatment liquid L on the substrate W. The solidified film G increases on the substrate W. The solidified film G grows on the substrate W. The treatment liquid L on the substrate W further decreases. The liquid film M further becomes thinner.

Finally, the solvent is entirely removed from the substrate W. The treatment liquid L entirely disappears from the substrate W. The liquid film M entirely disappears from the substrate W. The liquid is not present on the substrate W. The pattern P is not in contact with the liquid. The projection A is not in contact with the liquid.

The solidified film G covers the upper surface WS1 of the substrate W. The upper surface WS1 comes into contact with the solidified film G. The pattern P comes into contact with the solidified film G. The pattern P is supported by the solidified film G. The projection A comes into contact with the solidified film G. The projection A is supported by the solidified film G. The recess B is filled with the solidified film G. The recess B is entirely filled with only the solidified film G.

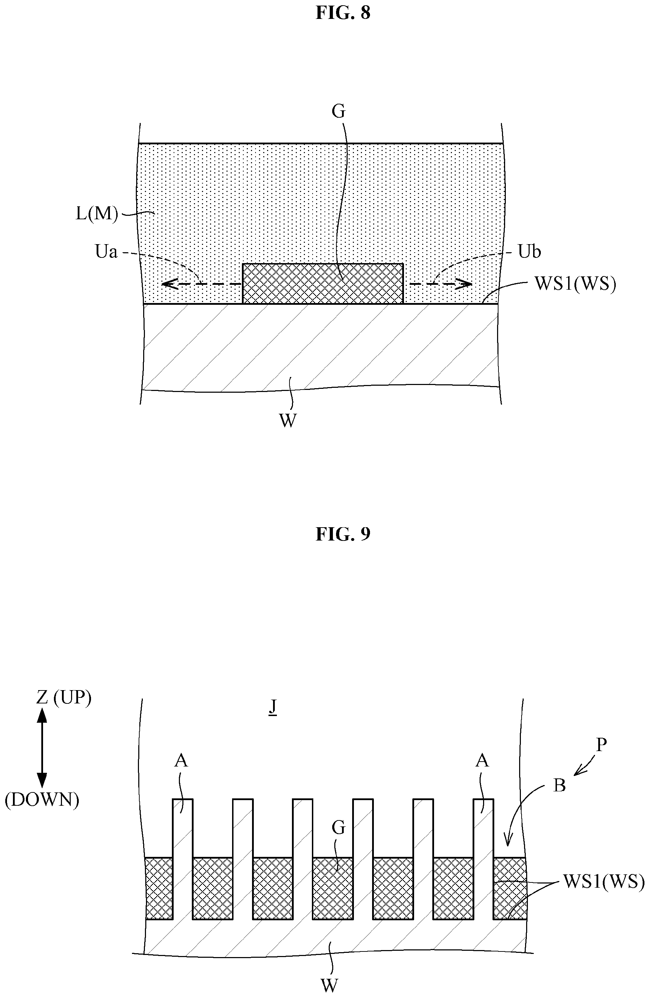

FIG. 8 is an enlarged view schematically illustrating the substrate W in the solidified film forming step. The growth of the solidified film G will be described again.

When the solidified film G grows, the solidified film G and the treatment liquid L are simultaneously present on the substrate W. The substrate W and the treatment liquid L come into contact with each other. The substrate W and the solidified film G come into contact with each other. The solidified film G and the treatment liquid L come into contact with each other. A first interface, a second interface, and a third interface are simultaneously formed. The first interface is an interface between the treatment liquid L and the substrate W. The second interface is an interface between the solidified film G and the substrate W. The third interface is an interface between the treatment liquid L and the solidified film G.

The first interface has the interfacial free energy $\gamma$LW. The second interface has the interfacial free energy $\gamma$GW. The third interface has the interfacial free energy $\gamma$LG.

Here, a coefficient K is defined by the interfacial free energies $\gamma$LW, $\gamma$GW, and $\gamma$LG. Specifically, the coefficient K is defined by Equation (1).

$$K = \gamma LG + \gamma GW - \gamma LW \;...(1)$$

The present inventors have found the following matters regarding the coefficient K. The lower the coefficient K is, the more likely the sublimable substance precipitates on the surface WS of the substrate W. The lower the coefficient K is, the more likely the solidified film G comes into contact with the surface WS. The lower the coefficient K is, the more likely the solidified film G spreads on the surface WS. The lower the coefficient K is, the more likely the solidified film G extends along the surface WS. For example, the lower the coefficient K is, the more likely the solidified film G extends in directions Ua and Ub on the surface WS. The coefficient K is an index indicating likeliness of spread of the solidified film G on the substrate W.

As described above, when the coefficient K is low, the solidified film G is likely to expand on the substrate W in the solidified film forming step. Accordingly, the solidified film G rapidly expands on the substrate W. The solidified film G rapidly expands also in the recess B. Rapid expansion of the solidified film G promotes evaporation of the solvent. Rapid expansion of the solidified film G promotes a decrease of the treatment liquid L. Thus, the solvent rapidly evaporates from the treatment liquid L on the substrate W. The treatment liquid L on the substrate W rapidly decreases. Therefore, the entire solvent is reliably removed from the substrate W by the end of the solidified film forming step. By the end of the solidified film forming step, the entire treatment liquid L on the substrate W surely disappears.

The controller 10 is configured to acquire the coefficient K. The controller 10 may acquire the coefficient K by various methods. The controller 10 reads the coefficient K, for example. The controller 10 selects the coefficient K, for example. The controller 10 calculates the coefficient K, for example. The controller 10 receives the coefficient K, for example.

The coefficient K is set in advance, for example. The coefficient K is set based on an experiment or analysis, for example. The coefficient K is stored in, for example, a storage medium of the controller 10. The controller 10 reads out, for example, the coefficient K stored in the storage medium.

A plurality of coefficients K is set depending on the composition of the treatment liquid L, for example. The plurality of coefficients K is set depending on the composition of a plurality of solidified films G, for example. The plurality of coefficients K is set depending on the state of the surface WS of the substrate W, for example. The plurality of coefficients K is set for each of a plurality of pieces of processing condition information, for example. The controller 10 may select one coefficient K from the plurality of coefficients K.

Here, the state of the surface WS of the substrate W is, for example, an affinity between the substrate W and water. The state of the surface WS is, for example, an affinity between the surface WS and water.

For example, the controller 10 may calculate the coefficient K based on the composition of the treatment liquid L. For example, the controller 10 may calculate the coefficient K based on the composition of the solidified film G. For example, the controller 10 may calculate the coefficient K based on the state of the surface WS of the substrate W. For example, the controller 10 may calculate the coefficient K based on the state of the surface WS of the substrate W in at least one of the treatment liquid supply step or the solidified film forming step. Here, the controller 10 may specify at least one of the composition of the treatment liquid L or the composition of the solidified film G based on the processing condition information. The controller 10 may specify the composition of the solidified film G based on the composition of the treatment liquid L. The controller 10 may estimate the state of the surface WS of the substrate W in at least one of the treatment liquid supply step or the solidified film forming step based on the composition of the chemical liquid and the composition of the cleaning liquid. The controller 10 may specify the composition of the chemical liquid and the composition of the cleaning liquid based on the processing condition information.

For example, the controller 10 may receive the coefficient K from an internal device of the substrate treating apparatus 1. The internal device of the substrate treating apparatus 1 is, for example, an input unit (not illustrated). A user may give the coefficient K to the controller 10 via the input unit. For example, the controller 10 may receive the coefficient K from an external device of the substrate treating apparatus 1. The external device of the substrate treating apparatus 1 is, for example, a host computer.

The controller 10 is configured to monitor the coefficient K. As a result, the quality of the treatment on the substrate W can be monitored.

The controller 10 is configured to manage the coefficient K. As a result, the quality of the treatment on the substrate W can be managed.

For example, the controller 10 controls the coefficient K to be equal to or less than a threshold TH.

Here, the threshold TH is set in advance, for example. The threshold TH is, for example, a constant. The threshold TH is set based on an experiment or analysis, for example.

The controller 10 is configured to acquire the threshold TH. The controller 10 may acquire the threshold TH by various methods. The controller 10 reads the threshold TH, for example. The controller 10 selects the threshold TH, for example. The controller 10 calculates the threshold TH, for example. The controller 10 receives the threshold TH, for example.

The threshold TH is set in advance, for example. The threshold TH is set based on an experiment or analysis, for example. The threshold TH is stored in, for example, the storage medium of the controller 10. The controller 10 reads, for example, the threshold TH stored in the storage medium.

Step S17: Sublimation step

The solidified film G sublimates. The substrate W is dried.

The supply unit 15f supplies a dry gas to the substrate W held by the substrate holder 13. The dry gas is supplied to the upper surface WS1 of the substrate W. The dry gas is supplied to the solidified film G. The solidified film G is exposed to the dry gas. Accordingly, the solidified film G smoothly sublimates. The solidified film G changes into a gas without passing through a liquid. The solidified film G is removed from the substrate W by sublimation of the solidified film G. Then, the supply unit 15f stops supplying the dry gas to the solidified film G.

FIG. 9 is a view schematically illustrating the substrate W in the sublimation step. As the solidified film G sublimates, the solidified film G decreases. As the solidified film G sublimates, the solidified film G becomes thinner.

The pattern P starts to be exposed to the gas J. The projection A starts to be exposed to the gas J. The gas J starts to enter the recess B.

When the solidified film G sublimates, the solidified film G does not change into a liquid. Accordingly, in the sublimation step, no liquid is generated on the substrate W. Thus, in the sublimation step, the pattern P does not receive a significant force. In the sublimation step, the projection A receives no significant force. The significant force is, for example, a surface tension of the liquid.

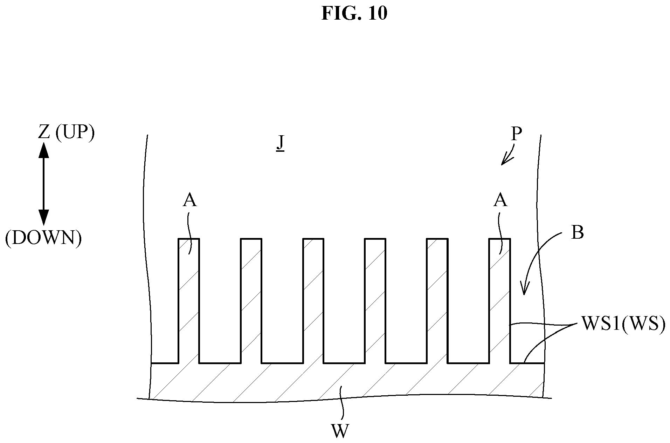

FIG. 10 is a view schematically illustrating the substrate W in the sublimation step. FIG. 10 schematically illustrates the substrate W at the end of the sublimation step, for example. Finally, the solidified film G is entirely removed from the substrate W. The liquid is not present on the substrate W. The pattern P is entirely exposed to the gas J. The entire projection A is exposed to the gas J. The entire recess B is filled with only the gas J. The substrate W is dried.

The treatment in the treatment liquid supply step, the solidified film forming step, and the sublimation step described above is an example of the dry treatment.

Step S18: Rotation stopping step

The substrate W stops rotating.

The rotation driving unit 14 stops rotating the substrate holder 13. The substrate W stops integrally with the substrate holder 13. The substrate W rests. The treating unit 11 ends the treatment on the substrate W.

<1-6. Second Operation Example of Supply Source 19a and Treating Unit 11>

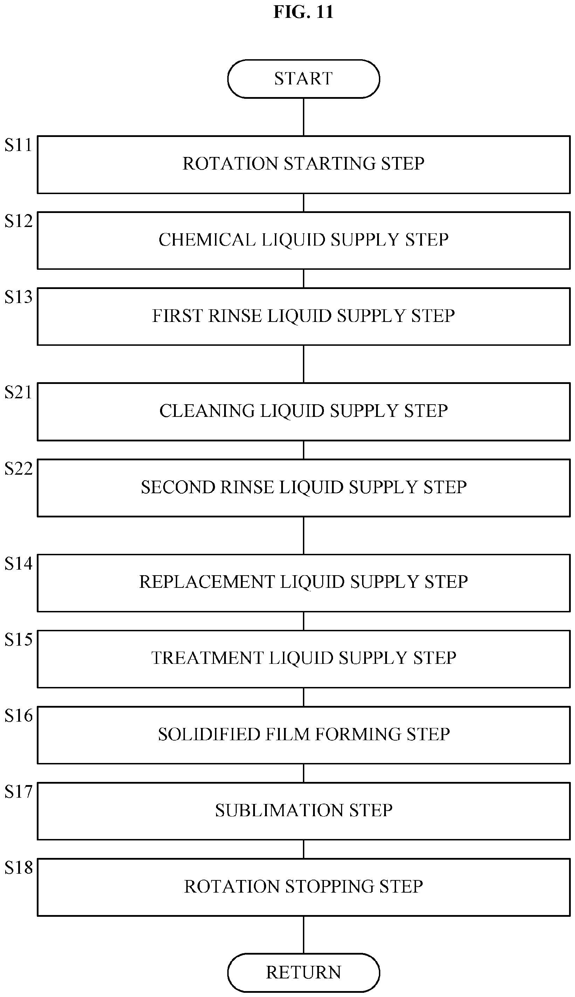

Reference is made to FIGS. 4 and 11. FIG. 11 is a flowchart showing procedures of a substrate treating method according to a first embodiment. FIG. 11 shows procedures of a second operation example. In the second operation example, the substrate treating method includes Step S1 and Steps S11 to S18. FIG. 11 omits illustration of Step S1, for convenience. In the second operation example, the substrate treating method further includes Steps S21 and S22. Steps S21 and S22 are executed after Step S13. Steps S21 and S22 are executed before Step S14.

The operations in Steps S1 and S11 to S18 are substantially common between the first operation example and the second operation example. Accordingly, description of operations in Steps S1, S11, and S17 to S18 is to be omitted. Operations in Steps S12, S13, S21, S22, S14, S15, and S16 will be described.

Step S12: Chemical liquid supply step

The chemical liquid is supplied to the substrate W. When the chemical liquid is hydrofluoric acid, the surface WS of the substrate W is modified to be hydrophobic.

Step S13: First rinse liquid supply step

The rinse liquid is supplied to the substrate W. Even after the first rinse liquid supply step, the surface WS of the substrate W has hydrophobicity.

Step S21: Cleaning liquid supply step

The cleaning liquid is supplied to the substrate W.

The supply unit 15d supplies the cleaning liquid to the substrate W. The cleaning liquid is supplied to the upper surface WS1 of the substrate W. The cleaning liquid removes the rinse liquid from the substrate W. The rinse liquid on the substrate W is replaced with the cleaning liquid. For example, the cleaning liquid cleans the substrate W. Then, the supply unit 15d stops supplying the cleaning liquid to the substrate W.

When the cleaning liquid is SC1, the cleaning liquid forms an oxide film on the upper surface WS1, and terminates the upper surface WS1 with a hydroxy group. For example, the hydroxy group is bonded to an atom (e.g. a silicon atom) located on the upper surface WS1 of the substrate W. Accordingly, the upper surface WS1 of the substrate W is modified to be hydrophilic. The upper surface WS1 of the substrate W is modified from hydrophobic to hydrophilic. For example, the affinity between the substrate W and water at the end of the cleaning liquid supply step is higher than the affinity between the substrate W and water at the start of the cleaning liquid supply step. For example, the affinity between the substrate W and water at the end of the cleaning liquid supply step is higher than the affinity between the substrate W and water at the end of the chemical liquid supply step.

Step S22: Second rinse liquid supply step

The rinse liquid is supplied to the substrate W.

The supply unit 15c supplies the rinse liquid to the substrate W held by the substrate holder 13. The rinse liquid is supplied to the upper surface WS1 of the substrate W. The rinse liquid removes the cleaning liquid from the substrate W. The cleaning liquid on the substrate W is replaced with the rinse liquid. Then, the supply unit 15c stops supplying the rinse liquid to the substrate W.

Even after the second rinse liquid supply step, the substrate W is still terminated with the hydroxy group. Thus, even after the second rinse liquid supply step, the substrate W has hydrophilicity.

Step S14: Replacement liquid supply step

The replacement liquid is supplied to the substrate W. Even after the replacement liquid supply step, the substrate W is still terminated with the hydroxy group. Accordingly, even after the replacement liquid supply step, the upper surface WS1 of the substrate W has hydrophilicity.

Step S15: Treatment liquid supply step

In the treatment liquid supply step, the upper surface WS1 of the substrate W has hydrophilicity. The treatment liquid L is supplied to the substrate W. Even after the treatment liquid supply step, the substrate W is still terminated with the hydroxy group. Accordingly, even after the treatment liquid supply step, the upper surface WS1 of the substrate W has hydrophilicity.

Step S16: Solidified film forming step

In the solidified film forming step, the upper surface WS1 of the substrate W has hydrophilicity. The solvent evaporates from the treatment liquid L on the substrate W. The solidified film G is formed on the substrate W.

As described above, in the second operation example, the upper surface WS1 of the substrate W changes from hydrophobic to hydrophilic. The affinity between the substrate W and water in the treatment liquid supply step of the second operation example is higher than the affinity between the substrate W and water in the treatment liquid supply step of the first operation example. The affinity between the substrate W and water in the solidified film forming step of the second operation example is higher than the affinity between the substrate W and water in the solidified film forming step of the first operation example.

<1-7. Technical Significance of Coefficient K>

Technical significance of the coefficient K will be described with reference to Examples 1a, 1b, 2a, 2b, 3a, 3b, 4a, and 4b. Hereinafter, the upper surface WS1 is called the surface WS, as appropriate.

Examples 1a, 1b, 2a, 2b, 3a, 3b, 4a, and 4b are different from each other with respect to the state of the surface WS of the substrate W and the composition of the treatment liquid.

Conditions of Example 1a will be described. In Example 1a, the substrate W is treated by the substrate treating method of the first operation example. Specifically, in Example 1a, a series of treatments including the chemical liquid supply step, the first rinse liquid supply step, the replacement liquid supply step, the treatment liquid supply step, the solidified film forming step, and the sublimation step is performed on the substrate W.

In the chemical liquid supply step, the chemical liquid is hydrofluoric acid. Hydrofluoric acid is a mixed liquid of hydrogen fluoride and deionized water. The volume ratio of hydrogen fluoride and deionized water is as follows.
Hydrogen fluoride : deionized water = 1 : 10 (volume ratio)

In the first rinse liquid supply step, the rinse liquid is deionized water.

In the replacement liquid supply step, the replacement liquid is isopropyl alcohol.

In the treatment liquid supply step, the treatment liquid is composed of a sublimable substance and a solvent. The sublimable substance is cyclohexanone oxime. The solvent is isopropyl alcohol. The volume ratio between the sublimable substance and the solvent is as follows.
Sublimable substance : solvent = 1 : 40 (volume ratio)

In the solidified film forming step, the substrate W is rotated at a rotation speed of 1500rpm.

In the sublimation step, the substrate W is rotated at a rotation speed of 1500rpm. Further, in the sublimation step, the dry gas is supplied to the substrate W. The dry gas is air.

In the treatment liquid supply step and the solidified film forming step of Example 1a, the surface WS of the substrate W has hydrophobicity. That is, the state of the surface WS in the treatment liquid supply step and the solidified film forming step is hydrophobic. The suffix "a" in Example 1a means that the state of the surface WS in the treatment liquid supply step and the solidified film forming step is hydrophobic.

Conditions of Example 1b will be described. In Example 1b, the substrate W is treated by the substrate treating method of the second operation example. Specifically, in Example 2, a series of treatments including the chemical liquid supply step, the first rinse liquid supply step, the cleaning liquid supply step, the second rinse liquid supply step, the replacement liquid supply step, the treatment liquid supply step, the solidified film forming step, and the sublimation step is performed on the substrate W.

In the cleaning liquid supply step, the cleaning liquid is SC1. SC1 is a mixed liquid of ammonia, hydrogen peroxide, and deionized water. The volume ratio of ammonia, hydrogen peroxide, and deionized water is as follows.

Ammonia : hydrogen peroxide : deionized water = 1 : 8 : 60 (volume ratio)

In the second rinse liquid supply step, the rinse liquid is deionized water.

The other conditions in Example 1b are the same as those in Example 1a.

In the treatment liquid supply step and the solidified film forming step of Example 2, the surface WS of the substrate W has hydrophilicity. That is, the state of the surface WS in the treatment liquid supply step and the solidified film forming step is hydrophilic. The suffix "b" in Example 1b means that the state of the surface WS in the treatment liquid supply step and the solidified film forming step is hydrophilic.

Conditions of Examples 2a and 2b will be described. In the treatment liquid supply step, the treatment liquid is composed of a sublimable substance and a solvent. The sublimable substance is cyclohexanone oxime. The solvent is methanol. The volume ratio between the sublimable substance and the solvent is as follows.

Sublimable substance : solvent = 1 : 40 (volume ratio)

The other conditions in Example 2a are the same as those in Example 1a. The other conditions in Example 2b are the same as those in Example 1b.

Conditions of Examples 3a and 3b will be described. In the treatment liquid supply step, the treatment liquid is composed of a sublimable substance and a solvent. The sublimable substance is camphor. The solvent is isopropyl alcohol. The volume ratio between the sublimable substance and the solvent is as follows.

Sublimable substance : solvent = 1 : 110 (volume ratio)

The other conditions in Example 3a are the same as those in Example 1a. The other conditions in Example 3b are the same as those in Example 1b.

Conditions of Examples 4a and 4b will be described. In the treatment liquid supply step, the treatment liquid is composed of a sublimable substance and a solvent. The sublimable substance is camphor. The solvent is methanol. The volume ratio between the sublimable substance and the solvent is as follows.

Sublimable substance : solvent = 1 : 100 (volume ratio)

The other conditions in Example 4a are the same as those in Example 1a. The other conditions in Example 4b are the same as those in Example 1b.

The substrates W treated in Examples 1a, 1b, 2a, 2b, 3a, 3b, 4a, and 4b were evaluated based on an average collapse rate E.

The average collapse rate E is determined as follows. The average collapse rate E is an average value of a plurality of local collapse rates $e_i$. The local collapse rate $e_i$ is a collapse rate in a local area $F_i$. i is any natural number from 1 to NF. The number NF is the number of local areas $F_i$. The number NF is a natural number of 2 or more. Each local area $F_i$ is a minute region of the substrate W. Each local area $F_i$ is magnified 50,000 times, for example, by a scanning electron microscope. An observer observes the pattern P (projections A) in each local area $F_i$. The observer observes the projections A in each local area $F_i$ one by one. Specifically, the observer classifies each of the projections A into either a collapsed projection A or a non-collapsed projection A. Here, the number of projections A observed in each local area $F_i$ is defined as $NA_i$. The number of collapsed projections A in each local area $F_i$ is defined as $NB_i$. The number $NB_i$ is equal to or less than the number $NA_i$. The local collapse rate $e_i$ is a ratio of the number $NB_i$ to the number $NA_i$. The local collapse rate $e_i$ is defined by, for example, the following equation.

$$e_i = NB_i/NA_i*100 \ (\%)$$

The average collapse rate E is a value obtained by dividing the sum of the local collapse rates $e_i$ by the number NF.

Furthermore, coefficients K in Examples 1a, 1b, 2a, 2b, 3a, 3b, 4a, and 4b were acquired.

An example of a procedure for acquiring the coefficient K will be described. The procedure for acquiring the coefficient K includes, for example, a preparation step, a measurement step, and a calculation step. The procedure for acquiring the coefficient K will be described using the coefficient K in Examples 3a and 3b as an example.

<<Preparation Step>>

As the substrate W, a first substrate Wa and a second substrate Wb are prepared. The first substrate Wa simulates the substrate W in the treatment liquid supply step of Example 3a. For example, the first substrate Wa is a substrate W subjected to a treatment including only the chemical liquid supply step, the first rinse liquid supply step, the replacement liquid supply step, and a spin drying step. Here, in the spin drying step, the substrate W is rotated to shake off the liquid (e.g. the replacement liquid) on the substrate W and the substrate W is dried. The surface WS of the first substrate Wa has hydrophobicity. The second substrate Wb simulates the substrate W in the treatment liquid supply step of Example 3b. For example, the second substrate Wb is a substrate W subjected to a treatment including only the chemical liquid supply step, the first rinse liquid supply step, the cleaning liquid supply step, the second rinse liquid supply step, the replacement liquid supply step, and the spin drying step. The surface WS of the second substrate Wb has hydrophilicity.

Further, the solidified film G and the treatment liquid L are prepared. The solidified film G simulates, for example, the solidified films G of Examples 3a and 3b. The solidified film G is made of, for example, solid camphor. The solidified film G prepared for obtaining the coefficient K need not be formed on the substrate W. The solidified film G has, for example, a block shape or a plate shape. The treatment liquid L is, for example, a treatment liquid used in Examples 3a and 3b. The treatment liquid L consists of, for example, camphor and isopropyl alcohol.

Furthermore, a first standard solution and a second standard solution are prepared. Here, regarding the first standard solution and the second standard solution, values of surface free energies $\gamma 1$ and $\gamma 2$, dispersive components $\gamma 1d$ and $\gamma 2d$, and polar components $\gamma 1p$ and $\gamma 2p$ are known.

$\gamma 1$ represents a surface free energy of the first standard solution.
$\gamma 1d$ represents a dispersive component of the surface free energy $\gamma 1$ of the first standard solution.
$\gamma 1p$ represents a polar component of the surface free energy $\gamma 1$ of the first standard solution.
$\gamma 2$ represents a surface free energy of the second standard solution.
$\gamma 2d$ represents a dispersive component of the surface free energy $\gamma 2$ of the second standard solution.
$\gamma 2p$ represents a polar component of the surface free energy $\gamma 2$ of the second standard solution.

The first standard solution is, for example, diiodomethane. The second standard solution is, for example, glycerin.

<<Measurement Step>>

Contact angles $\theta 1a$, $\theta 1b$, $\theta 2a$, $\theta 2b$, $\theta 3$, $\theta 4$, and $\theta 5$ were measured.

$\theta 1a$ represents a contact angle between the first standard solution and the first substrate Wa.
$\theta 1b$ represents a contact angle between the first standard solution and the second substrate Wb.
$\theta 2a$ represents a contact angle between second standard solution and first substrate Wa.
$\theta 2b$ represents a contact angle between the second standard solution and the second substrate Wb.
$\theta 3$ represents a contact angle between the first standard solution and the solidified film G.
$\theta 4$ represents a contact angle between the second standard solution and the solidified film G.
$\theta 5$ represents a contact angle between the treatment liquid L and the solidified film G.

Each of the contact angles $\theta 1a$, $\theta 1b$, $\theta 2a$, $\theta 2b$, $\theta 3$, $\theta 4$, and $\theta 5$ is measured by, for example, a contact angle method. Each of the contact angles $\theta 1a$, $\theta 1b$, $\theta 2a$, $\theta 2b$, $\theta 3$, $\theta 4$, and $\theta 5$ is measured using, for example, a contact angle meter. Each of the contact angles $\theta 1a$, $\theta 1b$, $\theta 2a$, $\theta 2b$, $\theta 3$, $\theta 4$, and $\theta 5$ is measured, for example, under an environment of normal temperature. Each of the contact angles $\theta 1a$, $\theta 1b$, $\theta 2a$, $\theta 2b$, $\theta 3$, $\theta 4$, and $\theta 5$ is measured, for example, under an environment of normal pressure.

Further, the surface free energy $\gamma L$ was measured.
$\gamma L$ represents a surface free energy of the treatment liquid L.

The surface free energy $\gamma L$ is measured, for example, by a pendant drop method. The surface free energy $\gamma L$ is measured, for example, under an environment of normal temperature. The surface free energy $\gamma L$ is measured, for example, under an environment of normal pressure.

<<Calculation Step>>

The coefficient K is calculated based on the values measured in the measurement step. Hereinafter, the coefficient K of Example 3a is calculated for convenience.

In the calculation of the coefficient K, Equations (2) to (4) are used in addition to Equation (1).

$$\gamma = \gamma d + \gamma p \;...(2)$$

$$\gamma AB = \gamma A - \gamma B \cos\theta \;...(3)$$

$$\gamma A + \gamma B - \gamma AB = 2(\gamma Ad \cdot \gamma Bd)^{1/2} + 2(\gamma Ap \cdot \gamma Bp)^{1/2} \;...(4)$$

A surface free energy $\gamma W$, a dispersive component $\gamma Wd$, and a polar component $\gamma Wp$ are calculated based on the contact angles $\theta 1a$ and $\theta 2a$. In this calculation, known surface free energies $\gamma 1$ and $\gamma 2$, dispersive components $\gamma 1d$ and $\gamma 2d$, and polar components $\gamma 1p$ and $\gamma 2p$ are used.

yW represents a surface free energy of the substrate W.
yWd represents a dispersive component of the surface free energy yW of the substrate W.
yWp represents a polar component of the surface free energy yW of the substrate W.

In this calculation, Equations (2), (3), and (4) are used. For example, Equation (5) is derived from Equations (3) and (4).

$$\gamma B(1 + \cos\theta) = 2(\gamma Ad \cdot \gamma Bd)^{1/2} + 2(\gamma Ap \cdot \gamma Bp)^{1/2} \;...(5)$$

In Equation (5), the surface free energy $\gamma 1$ is substituted for $\gamma B$, the dispersive component $\gamma 1d$ is substituted for yBd, the polar component $\gamma 1p$ is substituted for $\gamma Bp$, and the contact angle $\theta 1a$ is substituted for $\theta$. Thus, a first equation is established. In Equation (5), the surface free energy $\gamma 2$ is substituted for $\gamma B$, the dispersive component $\gamma 2d$ is substituted for yBd, the polar component $\gamma 2p$ is substituted for $\gamma Bp$, and the contact angle $\theta 2a$ is substituted for $\theta$. Thus, a second equation is established. The values of $\gamma Ad$ and $\gamma Ap$ each are calculated from the first equation and the second equation. The value of $\gamma Ad$ corresponds to the value of the dispersive component $\gamma Wd$. The value of $\gamma Ap$ corresponds to the value of the polar component $\gamma Wp$. Further, in Equation (2), the dispersive component yWd is substituted for $\gamma d$, and the polar component $\gamma Wp$ is substituted for $\gamma p$. Thus, the value of $\gamma$ is calculated. The value of $\gamma$ corresponds to the value of the surface free energy $\gamma W$. That is, the sum of the dispersive component yWd and the polar component $\gamma Wp$ is equal to the surface free energy yW.

A surface free energy $\gamma G$, a dispersive component $\gamma Gd$, and a polar component $\gamma Gp$ are calculated based on the contact angles $\theta 3$ and $\theta 4$. In this calculation, known surface free energies $\gamma 1$ and $\gamma 2$, dispersive components $\gamma 1d$ and $\gamma 2d$, and polar components $\gamma 1p$ and $\gamma 2p$ are used.

$\gamma G$ represents a surface free energy of the solidified film G.
$\gamma Gd$ represents a dispersive component of the surface free energy $\gamma G$ of the solidified film G.
$\gamma Gp$ represents a polar component of the surface free energy $\gamma G$ of the solidified film G.

In this calculation, Equations (2), (3), and (4) are used. The calculation of the surface free energy $\gamma G$, the dispersive component $\gamma Gd$, and the polar component $\gamma Gp$ is similar to the calculation of the surface free energy $\gamma W$, the dispersive component $\gamma Wd$, and the polar component $\gamma Wp$.

The interfacial free energy $\gamma GW$ is calculated. In this calculation, the calculated surface free energies $\gamma G$ and $\gamma W$, dispersive components $\gamma Gd$ and $\gamma Wd$, and polar components $\gamma Gp$ and $\gamma Wp$ are used.

In this calculation, Equation (4) is used. For example, in Equation (4), the surface free energies $\gamma G$ and $\gamma W$ are substituted for $\gamma A$ and $\gamma B$, respectively, the dispersive components $\gamma Gd$ and $\gamma Wd$ are substituted for $\gamma Ad$ and $\gamma Bd$, respectively, and the polar components $\gamma Gp$ and $\gamma Wp$ are substituted for $\gamma Ap$ and $\gamma Bp$, respectively. Thus, the value of $\gamma AB$ is calculated. The value of yAB corresponds to the value of the interfacial free energy yGW.

The interfacial free energy $\gamma LG$ is calculated based on the contact angle $\theta 5$ and the surface free energy $\gamma L$. In this calculation, the calculated surface free energy $\gamma G$ is used.

In this calculation, Equation (3) is used. For example, in Equation (3), the surface free energy $\gamma G$ is substituted for $\gamma A$, the surface free energy $\gamma L$ is substituted for $\gamma B$, and the contact angle $\theta 5$ is substituted for $\theta$. Thus, the value of $\gamma AB$ is calculated. The value of $\gamma AB$ corresponds to the value of the interfacial free energy $\gamma LG$.

A dispersive component $\gamma Ld$ and a polar component $\gamma Lp$ are calculated based on the surface free energy yL. In this calculation, the calculated surface free energy $\gamma G$, interfacial free energy yLG, dispersive component $\gamma Gd$, and polar component $\gamma Gp$ are used.

$\gamma Ld$ represents a dispersive component of the surface free energy $\gamma L$ of the treatment liquid L.

$\gamma$Lp represents a polar component of the surface free energy $\gamma$L of the treatment liquid L.

In this calculation, Equations (2) and (4) are used. In Equation (4), the surface free energy $\gamma$L is substituted for $\gamma$A, the surface free energy $\gamma$G is substituted for $\gamma$B, the interfacial free energy $\gamma$LG is substituted for $\gamma$AB, the dispersive component $\gamma$Gd is substituted for $\gamma$Bd, and the polar component $\gamma$Gp is substituted for $\gamma$Bp. Thus, a third equation is established. From Equation (2), $\gamma$Ad and $\gamma$Ap in the third equation have the following relationship:

$$\gamma Ad + \gamma Ap = \gamma L$$

Using this relationship, the values of yAd and yAp each are calculated from the third equation. The value of yAd corresponds to the value of the dispersive component yLd. The value of yAp corresponds to the value of the polar component yLp.

The interfacial free energy yLW is calculated based on the surface free energy yL. In this calculation, the calculated surface free energy yW, dispersive components yLd and yWd, and polar components yLp and yWp are used.

In this calculation, Equation (4) is used. The calculation of the interfacial free energy yLW is similar to the calculation of the interfacial free energy yGW.

Finally, the coefficient K is calculated. In this calculation, the calculated interfacial free energies yLG, yGW, and yLW are used. In this calculation, Equation (1) is used.

According to the above procedure, the coefficient K of Example 3a is acquired.

When the coefficient K of Example 3b is calculated, the "contact angle $\theta$1a" and the "contact angle $\theta$1b" in the above description are changed to a "contact angle $\theta$1b" and a "contact angle $\theta$2b", respectively.

When the contact angles $\theta$1a and $\theta$1b are not distinguished, the contact angles $\theta$1a and $\theta$1b are simply called "contact angle $\theta$1". When the contact angles $\theta$2a and $\theta$2b are not distinguished, the contact angles $\theta$2a and $\theta$2b are simply called "contact angle $\theta$2". The surface free energy $\gamma$W, the dispersive component $\gamma$Wd, and the polar component $\gamma$Wp are calculated based on the contact angles $\theta$1 and $\theta$2. The interfacial free energies $\gamma$GW and $\gamma$LW are calculated based on the surface free energy yW, the dispersive component yWd, and the polar component yWp. Therefore, the interfacial free energies yGW and yLW are set depending on the surface free energy yW of the substrate W. The interfacial free energies yGW and yLW are set depending on the contact angles $\theta$1 and $\theta$2.

Here, the contact angles $\theta$1 and $\theta$2 are indices indicating the state of the surface WS of the substrate W. Thus, the interfacial free energies $\gamma$GW and $\gamma$LW are set depending on the state of the surface WS of the substrate W.

For example, the contact angles $\theta$1 and $\theta$2 are indices indicating the state of the surface WS of the substrate W in the treatment liquid supply step. Thus, the interfacial free energies $\gamma$GW and $\gamma$LW are set based on the state of the surface WS of the substrate W in the treatment liquid supply step.

For example, the contact angles $\theta$1 and $\theta$2 are also indices indicating the state of the surface WS of the substrate W in the solidified film forming step. Thus, the interfacial free energies yGW and yLW are set based on the state of the surface WS of the substrate W in the solidified film forming step.

More specifically, description will be made. For convenience, the surface free energy yW of the first substrate Wa is called "first surface free energy yWa". The surface WS of the first substrate Wa has hydrophobicity. Accordingly, the first surface free energy yWa corresponds to the surface free energy yW of the substrate W when the surface WS has hydrophobicity. When the coefficient K of Example 3a is acquired, the interfacial free energy yGW and the interfacial free energy yLW are each set based on the first surface free energy yWa. Accordingly, when the surface WS of the substrate W has hydrophobicity in the treatment liquid supply step, the interfacial free energy yGW and the interfacial free energy yLW are each set based on the first surface free energy yWa. When the surface WS of the substrate W has hydrophobicity in the solidified film forming step, the interfacial free energy yGW and the interfacial free energy yLW are each set based on the first surface free energy yWa.

For convenience, the surface free energy yW of the second substrate Wb is called "second surface free energy yWb". The surface WS of the second substrate Wb has hydrophilicity. The second surface free energy yWb corresponds to the surface free energy yW of the substrate W when the surface WS has hydrophilicity. When the coefficient K of Example 3b is acquired, the interfacial free energy yGW and the interfacial free energy yLW are each set based on the second surface free energy yWb. Accordingly, when the surface WS of the substrate W has hydrophilicity in the treatment liquid supply step, the interfacial free energy yGW and the interfacial free energy yLW are each set based on the second surface free energy yWb. When the surface WS of the substrate W has hydrophilicity in the solidified film forming step, the interfacial free energy yGW and the interfacial free energy yLW are each set based on the second surface free energy yWb.

The coefficient K is calculated based on the interfacial free energies yGW and yLW. As described above, the interfacial free energies $\gamma$GW and $\gamma$LW are set depending on the contact angles $\theta$1 and $\theta$2. The contact angles $\theta$1 and $\theta$2 are indices indicating the state of the surface WS of the substrate W. Thus, the coefficient K is set depending on the state of the surface WS of the substrate W. When the coefficient K is acquired, the state of the surface WS of the substrate W is taken into account.

For example, the coefficient K is set based on the state of the surface WS of the substrate W in the treatment liquid supply step. For example, the coefficient K is set based on the state of the surface WS of the substrate W in the solidified film forming step.

The coefficient K is calculated based on the interfacial free energies yGW and yLW. As described above, the interfacial free energies yGW and yLW are set depending on the surface free energy yW of the substrate W. Thus, the coefficient K is set depending on the surface free energy yW of the substrate W. For example, when the coefficient K of Example 3a is acquired, the coefficient K is set based on the first surface free energy yWa. Accordingly, when the surface WS of the substrate W has hydrophobicity in the treatment liquid supply step, the coefficient K is set based on the first surface free energy yWa. When the surface WS of the substrate W has hydrophobicity in the solidified film forming step, the coefficient K is set based on the first surface free energy yWa. For example, when the coefficient K of Example 3b is acquired, the coefficient K is set based on the second surface free energy yWb. Accordingly, when the surface WS of the substrate W has hydrophilicity in the treatment liquid supply step, the coefficient K is set based on the second surface free energy yWb. When the surface WS of the substrate W has hydrophilicity in the solidified film forming step, the coefficient K is set based on the second surface free energy yWb.

More specifically, the coefficient K includes the coefficient K of Example 3a and the coefficient K of Example 3b. For convenience, the coefficient K of Example 3a is called "first coefficient Ka". The coefficient K of Example 3b is called "second coefficient Kb". The first coefficient Ka corresponds to the coefficient K when the surface WS of the substrate W has hydrophobicity. The second coefficient Kb corresponds to the coefficient K when the surface WS of the substrate W has hydrophilicity. The coefficient K is switched between the first coefficient Ka and the second coefficient Kb depending on the state of the surface WS of the substrate W. Specifically, when the surface WS of the substrate W has hydrophobicity, the coefficient K is set to the first coefficient Ka. For example, when the surface WS of the substrate W has hydrophobicity in the treatment liquid supply step, the coefficient K is set to the first coefficient Ka. For example, when the surface WS of the substrate W has hydrophobicity in the solidified film forming step, the coefficient K is set to the first coefficient Ka. Meanwhile, when the surface WS of the substrate W has hydrophilicity, the coefficient K is set to the second coefficient Kb. For example, when the surface WS of the substrate W has hydrophilicity in the treatment liquid supply step, the coefficient K is set to the second coefficient Kb. For example, when the surface WS of the substrate W has hydrophilicity in the solidified film forming step, the coefficient K is set to the second coefficient Kb.

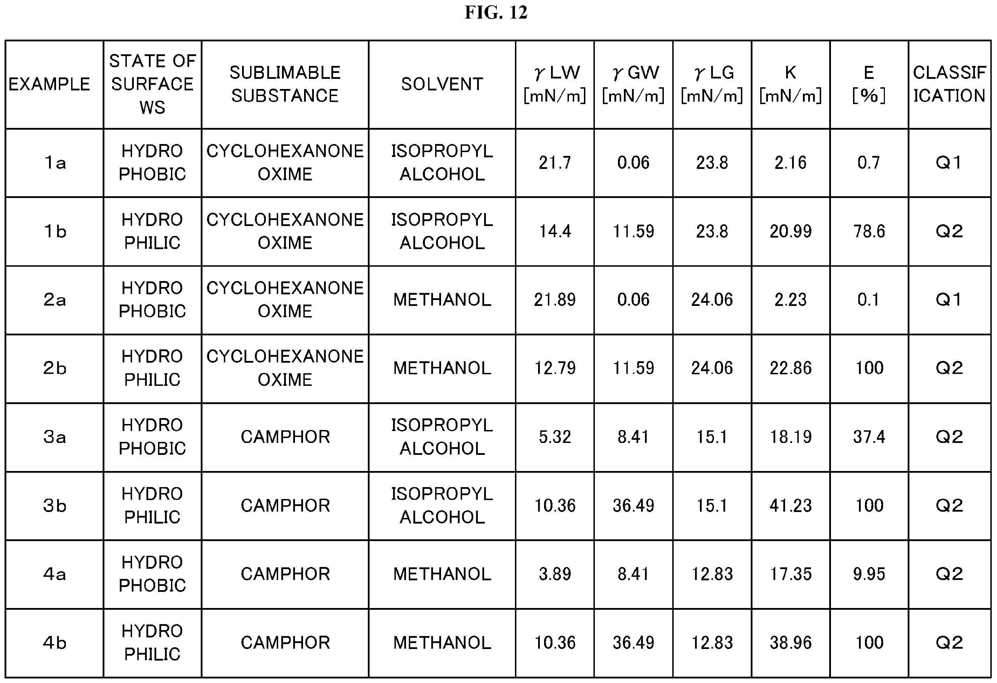

FIG. 12 is a table illustrating the coefficients K in Examples. The table of FIG. 12 further shows the interfacial free energies yLW, yGW, and yLG in Examples. The table of FIG. 12 further shows the average collapse rates E in Examples.

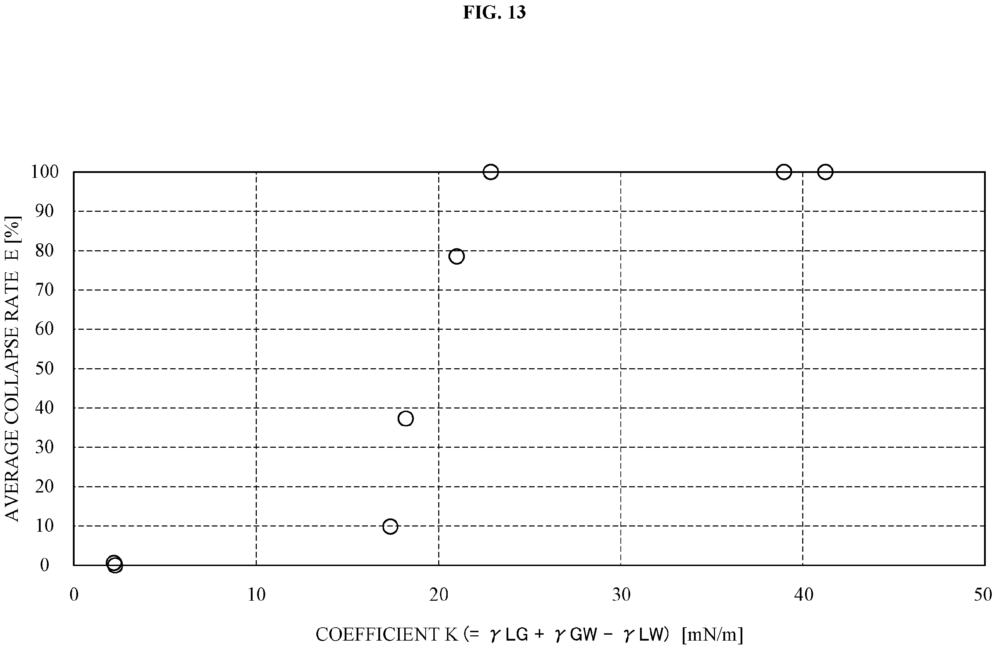

FIG. 13 is a graph showing the relationship between the coefficients K and the average collapse rates E in Examples.

The coefficient K has a value from 2.16 mN/m to 41.23 mN/m. Note that "mN/m" may be described as "$10^{-3}$N/m". When the coefficient K is 22.86 mN/m or more, the average collapse rate E is 100%. When the coefficient K is 20.99 mN/m or less, the average collapse rate E is 78.6% or less. When the coefficient K is 18.19 mN/m or less, the average collapse rate E is 37.4% or less. When the coefficient K is 17.35 mN/m or less, the average collapse rate E is 9.95% or less. When the coefficient K is 2.23 mN/m or less, the average collapse rate E is 0.7% or less.

The following is found from Examples 1a, 1b, 2a, 2b, 3a, 3b, 4a, and 4b. As the coefficient K decreases, the average collapse rate E decreases. This fact supports the matters that the present inventors have found regarding the coefficient K. For example, the fact indicates that "the lower the coefficient K is, the more likely the solidified film G expands on the substrate W in the solidified film forming step".

When the coefficient K decreases from 23 mN/m to 17 mN/m, the average collapse rate E decreases significantly.

When the coefficient K is 18 mN/m or less, the average collapse rate E is 40% or less. When the coefficient K is 18 mN/m or less, the substrate W is treated with an appropriate quality.

When the coefficient K is 17 mN/m or less, the average collapse rate E is 10% or less. When the coefficient K is 17 mN/m or less, the substrate W is treated with a more appropriate quality.

When the coefficient K is equal to or less than the threshold TH, the average collapse rate E is appropriately reduced. The threshold TH is preferably, for example, 18 mN/m. The threshold TH is preferably, for example, 17 mN/m.

As described above, the coefficient K and the average collapse rate E have a constant relationship with each other. It is possible to estimate the average collapse rate E from the coefficient K without actually measuring the average collapse rate E.

When the coefficient K is 23 mN/m or more, the pattern P collapses entirely. The reason for this is presumed as follows.

When the coefficient K is high, the sublimable substance is less likely to precipitate on the surface WS of the substrate W. For example, the sublimable substance is likely to precipitate in the bulk portion of the treatment liquid L. Here, the bulk portion is a portion of the treatment liquid L that is not in contact with the substrate W. The bulk portion is a portion of the treatment liquid L inward from an interface between the substrate W and the treatment liquid L. The bulk portion does not include the interface between the substrate W and the treatment liquid L. Thus, the solidified film G is less likely to come into contact with the surface WS of the substrate W. The solidified film G is less likely to spread on the surface WS. The solidified film G is less likely to extend along the surface WS.

As described above, when the coefficient K is high, the solidified film G is unlikely to expand on the substrate W in the solidified film forming step. Accordingly, the solidified film G does not rapidly expand on the substrate W. The solidified film G does not rapidly expand even in the recess B. Thus, the solidified film G does not promote evaporation of the solvent. The solidified film G does not promote a decrease of the treatment liquid L. Therefore, the solvent does not rapidly evaporate from the treatment liquid L on the substrate W. The treatment liquid L on the substrate W does not decrease rapidly. As a result, even at the end of the solidified film forming step, the solvent still remains on the substrate W. Even at the end of the solidified film forming step, the treatment liquid L still remains on the substrate W.

In the sublimation step, not only the solidified film G but also the treatment liquid L is present on the substrate W. Even after the solidified film G is entirely sublimated in the sublimation step, the treatment liquid L still remains on the substrate W. After the solidified film G is entirely sublimated, the pattern P receives a significant force from the treatment liquid L in a state where the pattern P is not supported by the solidified film G. The significant force is, for example, a surface tension of the treatment liquid L. As a result, the pattern P collapses.

<1-8. Effect of First Embodiment>

The substrate treating method is to treat a substrate W on which a pattern P is formed. The substrate treating method includes a treatment liquid supply step, a solidified film forming step, and a sublimation step. In the treatment liquid supply step, the treatment liquid L is supplied to the substrate W. The treatment liquid L contains a sublimable substance and a solvent. In the solidified film forming step, the solvent is evaporated from the treatment liquid L on the substrate W. In the solidified film forming step, the solidified film G is formed on the substrate W. The solidified film G contains the sublimable substance. In the sublimation step, the solidified film G is sublimated. The substrate W is dried by sublimation of the solidified film G.

The coefficient K is defined by Equation (1). The coefficient K is equal to or less than the threshold TH. The fact that the coefficient K is equal to or less than the threshold TH is called "first condition", as appropriate. The substrate W, the treatment liquid L, and the solidified film G satisfy the first condition. Accordingly, in the solidified film forming step, the solidified film G is suitably formed on the substrate W. Specifically, in the solidified film forming step, the solidified film G smoothly expands on the substrate W. Thus, in the solidified film forming step, the solvent is efficiently removed from the substrate W. In the solidified film forming step, the treatment liquid L efficiently disappears from the substrate W. Therefore, in the sublimation step, the substrate W is dried in a state where the pattern P formed on the substrate W is suitably protected.

As described above, according to the substrate treating method according to the first embodiment, the substrate is appropriately treated.

The threshold TH is a preset constant. Accordingly, the first condition is simple. Thus, it is easy to manage the substrate treating method so as to satisfy the first condition. For example, it is easy to manage the state of the surface WS of the substrate W in the treatment liquid supply step so as to satisfy the first condition. For example, it is easy to manage the composition of the treatment liquid L and the composition of the solidified film G so as to satisfy the first condition. Therefore, it is easy to execute the substrate treating method.

The threshold TH is, for example, 17 mN/m. In other words, the coefficient K is 17 mN/m or less. Accordingly, in the solidified film forming step, the solidified film G more smoothly expands on the substrate W. Thus, the pattern P is suitably protected by the solidified film G. Therefore, collapse of the pattern P is suitably suppressed.

The coefficient K is set depending on the state of the surface WS of the substrate W. Accordingly, the coefficient K is appropriately set regardless of the state of the surface WS of the substrate W. Thus, the substrate W is appropriately treated regardless of the state of the surface WS of the substrate W.

The coefficient K is set based on the state of the surface WS of the substrate W in the treatment liquid supply step. Accordingly, the coefficient K is appropriately set regardless of the state of the surface WS of the substrate W in the treatment liquid supply step. Thus, the substrate W is appropriately treated regardless of the state of the surface WS of the substrate W in the treatment liquid supply step.

The coefficient K is set based on the state of the surface WS of the substrate W in the solidified film forming step. Accordingly, the coefficient K is appropriately set regardless of the state of the surface WS of the substrate W in the solidified film forming step. Thus, the substrate W is appropriately treated regardless of the state of the surface WS of the substrate W in the solidified film forming step.

The interfacial free energy $\gamma GW$ and the interfacial free energy $\gamma LW$ are each set depending on the state of the surface WS of the substrate W. Accordingly, the interfacial free energy $\gamma GW$ and the interfacial free energy $\gamma LW$ are each appropriately set regardless of the state of the surface WS of the substrate W. Thus, when the coefficient K is set, the state of the surface WS of the substrate W is suitably taken into account. Therefore, the coefficient K is appropriately set regardless of the state of the surface WS of the substrate W.

The interfacial free energy yGW and the interfacial free energy yLW are each set based on the state of the surface WS of the substrate W in the treatment liquid supply step. Accordingly, the interfacial free energy yGW and the interfacial free energy yLW are each appropriately set regardless of the state of the surface WS of the substrate W in the treatment liquid supply step. Thus, the coefficient K is appropriately set regardless of the state of the surface WS of the substrate W in the treatment liquid supply step.

The interfacial free energy yGW and the interfacial free energy yLW are each set based on the state of the surface WS of the substrate W in the solidified film forming step. Accordingly, the interfacial free energy yGW and the interfacial free energy yLW are each appropriately set regardless of the state of the surface WS of the substrate W in the solidified film forming step. Thus, the coefficient K is appropriately set regardless of the state of the surface WS of the substrate W in the solidified film forming step.

When the surface WS of the substrate W has hydrophobicity in the treatment liquid supply step, the interfacial free energy yGW and the interfacial free energy yLW are each set based on the first surface free energy yWa. Accordingly, when the surface WS of the substrate W has hydrophobicity in the treatment liquid supply step, the interfacial free energy yGW and the interfacial free energy yLW are each appropriately set.

When the surface WS of the substrate W has hydrophilicity in the treatment liquid supply step, the interfacial free energy yGW and the interfacial free energy yLW are each set based on the second surface free energy yWb. Accordingly, when the surface WS of the substrate W has hydrophilicity in the treatment liquid supply step, the interfacial free energy yGW and the interfacial free energy yLW are each appropriately set.

In summary, the interfacial free energy yGW and the interfacial free energy yLW are each appropriately set regardless of the state of the surface WS of the substrate W in the treatment liquid supply step.

When the surface WS of the substrate W has hydrophobicity in the solidified film forming step, the interfacial free energy yGW and the interfacial free energy yLW are each set based on the first surface free energy yWa. Accordingly, when the surface WS of the substrate W has hydrophobicity in the solidified film forming step, the interfacial free energy yGW and the interfacial free energy yLW are each appropriately set.

When the surface WS of the substrate W has hydrophilicity in the solidified film forming step, the interfacial free energy yGW and the interfacial free energy yLW are each set based on the second surface free energy yWb. Accordingly, when the surface WS of the substrate W has hydrophilicity in the solidified film forming step, the interfacial free energy yGW and the interfacial free energy yLW are each appropriately set.

In summary, the interfacial free energy yGW and the interfacial free energy yLW are each appropriately set regardless of the state of the surface WS of the substrate W in the solidified film forming step.

<2. Second Embodiment>

A second embodiment will be described with reference to the drawings. Like numerals are used to identify like components which are the same as those in the first embodiment, and the components will not particularly be described.

Regarding the outline of the substrate treating apparatus 1, the construction of the treating unit 11, and the construction of the supply source 19a, the second embodiment is substantially the same as the first embodiment. Hereinafter, an operation example of the treating unit 11 according to the second embodiment will be described.

<2-1. Operation Example of Treating Unit 11>

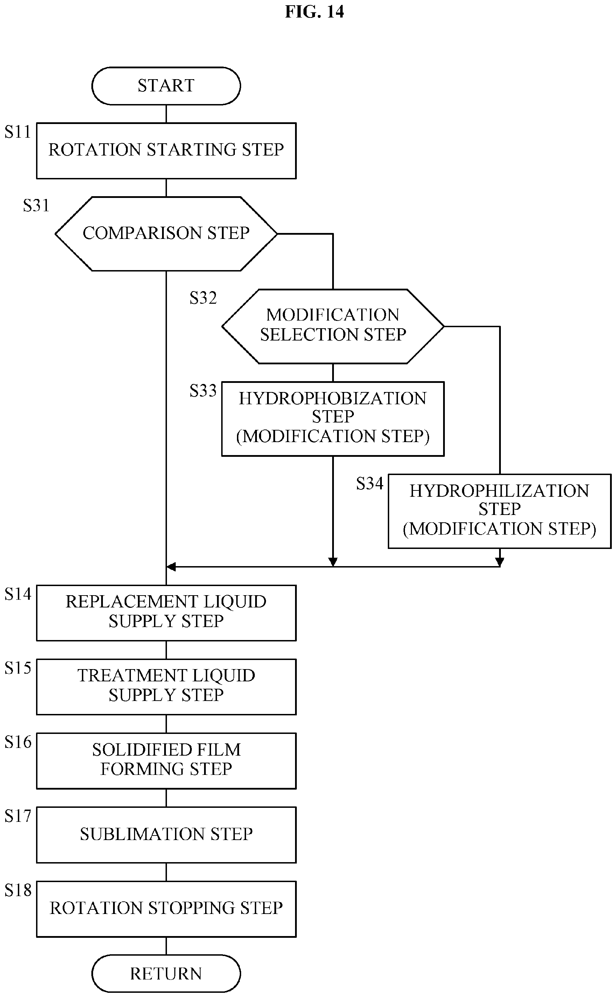

FIG. 14 is a flowchart showing procedures of the substrate treating method according to the second embodiment. The substrate treating method according to the second embodiment includes Steps S1, S11, and S14 to S18. FIG. 14 omits illustration of Step S1 described in the first embodiment, for convenience.

Further, the substrate treating method according to the second embodiment includes Steps S31 to S34. Steps S31 to S34 are executed after Step S11, for example. Steps S31 to S34 are executed before Step S14, for example. Step S31 is a comparison step. Step S32 is a modification selection step. Step S33 is a hydrophobization step. Step S34 is a hydrophilization step.

The hydrophobization step corresponds to the modification step in the present invention. The hydrophilization step corresponds to the modification step in the present invention. When the hydrophobization step and the hydrophilization step are not distinguished, the hydrophobization step and the hydrophilization step are collectively referred to as "modification step".

The operations in Steps S1, S11, and S14 to S18 are substantially common between the first embodiment and the second embodiment. Accordingly, description of operations in Steps S1, S11, and S14 to S18 is to be omitted. Operations in Steps S31 to S34 will be described.

Step S31: Comparison step

The coefficient K is compared with the threshold TH.

The coefficient K is defined by Equation (1).

More specifically, the coefficient K to be compared with the threshold TH is the coefficient K when the treatment liquid supply step and the solidified film forming step are performed without performing the modification step. Hereinafter, the coefficient K when the modification step is not performed before the treatment liquid supply step is called "coefficient Ki". The coefficient Ki may be called "initial coefficient Ki".

The coefficient Ki is set depending on the state of the surface WS of the substrate W. For example, the coefficient Ki is set based on the state of the surface WS of the substrate W in the treatment liquid supply step when the modification step is not performed before the treatment liquid supply step. For example, the coefficient Ki is set based on the state of the surface WS of the substrate W in the solidified film forming step when the modification step is not performed before the treatment liquid supply step.

The controller 10 is configured to acquire the coefficient Ki. The controller 10 may acquire the coefficient K by various methods. The controller 10 reads the coefficient Ki, for example. The controller 10 selects the coefficient Ki, for example. The controller 10 calculates the coefficient Ki, for example. The controller 10 receives the coefficient Ki, for example.

For example, the controller 10 has information regarding the coefficient K. The information regarding the coefficient K is, for example, the table shown in FIG. 12. The information regarding the coefficient K is stored in, for example, a storage medium of the controller 10. The controller 10 specifies the coefficient Ki from the information regarding the coefficient K, for example. The controller 10 selects the coefficient Ki from the information regarding the coefficient K, for example.

For example, the controller 10 acquires the coefficient Ki based on the state of the surface WS of the substrate W in the treatment liquid supply step when the modification step is not performed before the treatment liquid supply step. For example, the controller 10 acquires the coefficient Ki based on the state of the surface WS of the substrate W in the solidified film forming step when the modification step is not performed before the treatment liquid supply step.

The controller 10 is configured to acquire the threshold TH. The controller 10 may acquire the threshold TH by various methods.

The controller 10 compares the coefficient Ki with the threshold TH. When the coefficient Ki is larger than the threshold TH, the controller 10 determines to perform the modification step. When the coefficient Ki is larger than the threshold TH, the process proceeds to Step S32. When the coefficient Ki is equal to or less than the threshold TH, the controller 10 determines to skip the modification step. When the coefficient Ki is equal to or less than the threshold TH, the process proceeds to Step S14. When the coefficient Ki is equal to or less than the threshold TH, the modification step is not performed.

Step S32: Modification selection step

The content of the modification step is determined. The content of the modification step includes hydrophobization and hydrophilization. Either hydrophobization or hydrophilization is selected.

The controller 10 determines the modification step to be either hydrophobization or hydrophilization. When hydrophobization is selected, the process proceeds to Step S33. When hydrophilization is selected, the process proceeds to Step S34.

For example, the controller 10 determines the modification step based on the information regarding the coefficient K.

Here, the controller 10 preferably designates the modification step of decreasing the coefficient K. Preferably, the controller 10 designates the modification step of reducing the coefficient K below the coefficient Ki. Preferably, the controller 10 designates the modification step of decreasing the coefficient K to a value lower than the threshold TH.

Step S33: Hydrophobization step

The surface WS of the substrate W is modified to be hydrophobic.

The supply unit 15b supplies hydrofluoric acid to the substrate W.

Hydrofluoric acid modifies the surface WS of the substrate W to be hydrophobic.

The hydrophobization step is substantially the same as the chemical liquid supply step described in the first embodiment, for example.

Here, the coefficient K is preferably reduced by the hydrophobization step.

Specifically, a coefficient Km is preferably lower than the coefficient Ki. Here, the coefficient Km is the coefficient K when the modification step is performed before the treatment liquid supply step. The coefficient Km is the coefficient K when the treatment liquid supply step and the solidified film forming step are performed after the modification step. The coefficient Km is set based on, for example, the state of the surface WS of the substrate W in the treatment liquid supply step when the modification step is performed before the treatment liquid supply step. The coefficient Km is set based on, for example, the state of the surface WS of the substrate W in the solidified film forming step when the modification step is performed before the treatment liquid supply step. The coefficient Km may be called "correction coefficient Km".

More preferably, the coefficient K is decreased to a value lower than the threshold TH by the hydrophobization step. Specifically, the coefficient Km is preferably smaller than the threshold TH.

Step S34: Hydrophilization step

The surface WS of the substrate W is modified to be hydrophilic.

The supply unit 15c supplies SC1 to the substrate W. SC1 modifies the surface WS of the substrate W to be hydrophilic.

The hydrophilization step is substantially the same as the cleaning liquid supply step described in the first embodiment.

Here, the coefficient K is preferably reduced by the hydrophilization step. Specifically, a coefficient Km is preferably lower than the coefficient Ki.

More preferably, the coefficient K is decreased to a value lower than the threshold TH by the hydrophilization step. Specifically, the coefficient Km is preferably lower than the threshold TH.

<2-2. Effect of Second Embodiment>

The substrate treating method is to treat a substrate W on which a pattern P is formed. The substrate treating method includes a treatment liquid supply step, a solidified film forming step, and a sublimation step. In the treatment liquid supply step, the treatment liquid L is supplied to the substrate W. The treatment liquid L contains a sublimable substance and a solvent. In the solidified film forming step, the solvent is evaporated from the treatment liquid L on the substrate W. In the solidified film forming step, the solidified film G is formed on the substrate W. The solidified film G contains the sublimable substance. In the sublimation step, the solidified film G is sublimated. The substrate W is dried by sublimation of the solidified film G.

Further, the substrate treating method includes a modification step. The modification step is executed when the coefficient Ki is larger than the threshold TH. The modification step is executed before the treatment liquid supply step. The modification step modifies the surface WS of the substrate W. The modification step changes the coefficient Ki to the coefficient Km. When the coefficient Ki is larger than the threshold TH, the surface WS of the substrate W is modified in the modification step, and then the treatment liquid L is supplied to the substrate W in the treatment liquid supply step. Thus, even when the coefficient Ki is larger than the threshold TH, the solidified film G is suitably formed on the substrate W in the solidified film forming step. Specifically, in the solidified film forming step, the solidified film G smoothly expands on the substrate W. Therefore, in the solidified film forming step, the solvent is efficiently removed from the substrate W. In the solidified film forming step, the treatment liquid L efficiently disappears from the substrate W. Therefore, in the sublimation step, the substrate is dried in a state where the pattern P formed on the substrate W is suitably protected.

As described above, according to the substrate treating method according to the second embodiment, the substrate W is appropriately treated.

The coefficient K is reduced by the modification step. Specifically, the coefficient Km is lower than the coefficient Ki. Accordingly, in the solidified film forming step, the solidified film G is more suitably formed on the substrate W.

The coefficient K is decreased to a value lower than the threshold TH by the modification step. Accordingly, in the solidified film forming step, the solidified film G is more suitably formed on the substrate W.

In the modification step, either the hydrophobization step or the hydrophilization step is executed. In the modification step, the surface WS of the substrate W is modified to be either hydrophilic or hydrophobic. Accordingly, in the modification step, the surface WS of the substrate W can be flexibly modified. Thus, in the solidified film forming step, the solidified film G is more suitably formed on the substrate W.

In the hydrophobization step, the surface WS of the substrate W is modified to be hydrophobic. Accordingly, in the modification step, the surface WS of the substrate W can be suitably modified. Thus, in the solidified film forming step, the solidified film G is more suitably formed on the substrate W.

In the hydrophilization step, the surface WS of the substrate W is modified to be hydrophilic. Accordingly, in the modification step, the surface WS of the substrate W can be suitably modified. Thus, in the solidified film forming step, the solidified film G is more suitably formed on the substrate W.

When the coefficient Ki is equal to or less than the threshold TH, the modification step is not executed. Specifically, when the coefficient Ki is equal to or less than the threshold TH, the hydrophobization step is not executed, and the hydrophilization step is not executed. Accordingly, the time required for the substrate treating method can be effectively shortened. When the coefficient Ki is equal to or less than the threshold TH, the solidified film G is suitably formed on the substrate W in the solidified film forming step even when the modification step is not executed. Thus, even when the coefficient Ki is equal to or less than the threshold TH, the substrate W is appropriately treated.

<3. Third Embodiment>

A third embodiment will be described with reference to the drawings. Like numerals are used to identify like components which are the same as those in the first embodiment, and the components will not particularly be described.
Regarding the outline of the substrate treating apparatus 1, the third embodiment is substantially the same as the first embodiment. Hereinafter, the construction of the treating unit 11 of the third embodiment will be described.

<3-1. Construction of Treating Unit 11>

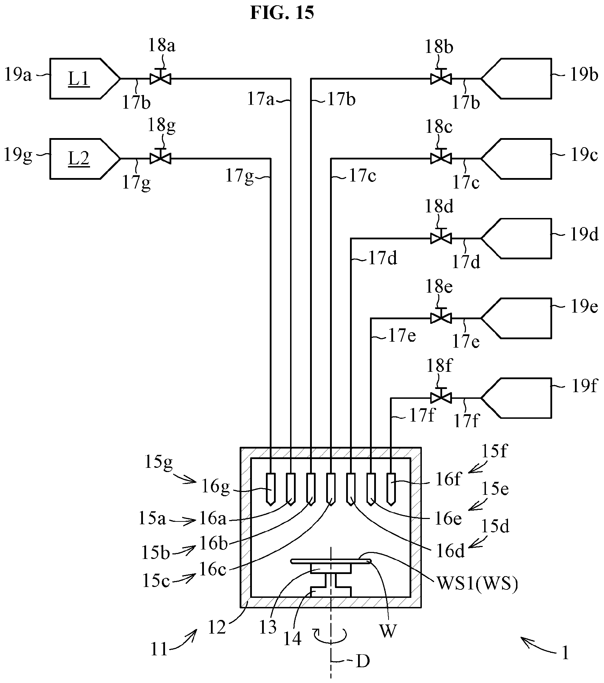

FIG. 15 is a diagram illustrating the construction of the treating unit 11 according to the third embodiment. FIG. 15 schematically shows the supply source 19a, for convenience.
The treating unit 11 includes a supply unit 15g, in addition to the supply units 15a to 15f. The supply unit 15g supplies the treatment liquid L to the substrate W held by the substrate holder 13.
Here, the composition of the treatment liquid L supplied by the supply unit 15g is different from the composition of the treatment liquid L supplied by the supply unit 15a. For convenience, the treatment liquid L supplied by the supply unit 15a is called "treatment liquid L1". The treatment liquid L supplied by the supply unit 15g is called "treatment liquid L2".
For example, the treatment liquid L1 and the treatment liquid L2 are different in terms of the composition of the sublimable substance. For example, the treatment liquid L1 and the treatment liquid L2 are different in terms of the composition of the solvent. For example, the treatment liquid L1 and the treatment liquid L2 are different in terms of the mixing ratio of the sublimable substance and the solvent.
The supply unit 15g includes a nozzle 16g, a pipe 17g, and a valve 18g. The nozzle 16g is installed in the interior of the casing 12. The nozzle 16g dispenses the treatment liquid L2 to the substrate W. The pipe 17g is connected to the nozzle 16g. The valve 18g is provided on the pipe 17g. The valve 18g controls the supply of the treatment liquid L2.
The supply unit 15g is connected to a supply source 19g. The supply source 19g is connected to, for example, the pipe 17g. The supply source 19g feeds the treatment liquid L2 to the supply unit 15g.

<3-2. Operation Example of Treating Unit 11>

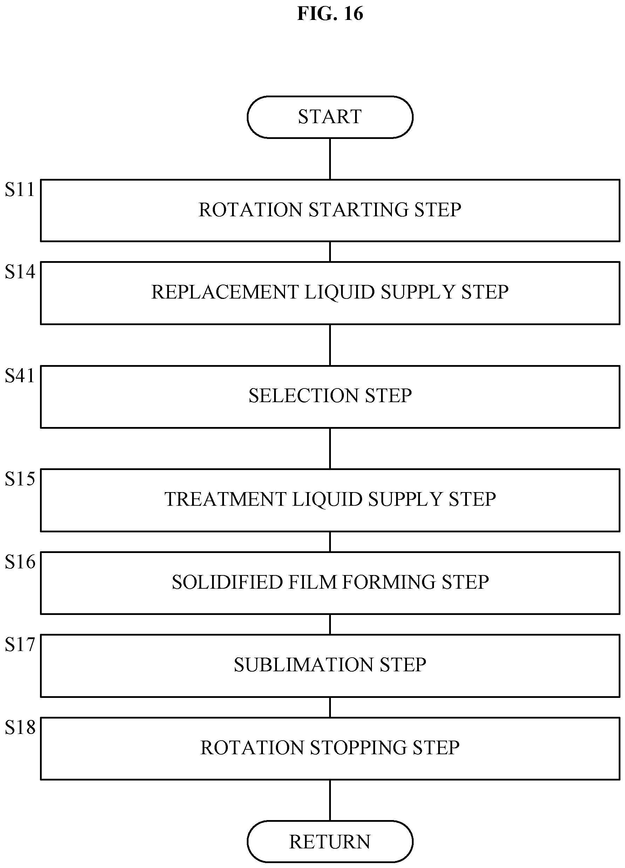

FIG. 16 is a flowchart showing procedures of the substrate treating method according to the third embodiment. The substrate treating method includes Steps S1, S11, and S14 to S18. Note that FIG. 16 omits illustration of Step S1, for convenience. The substrate treating method further includes Step S41. Step S41 is executed after Step S14, for example. Step S41 is executed before Step S15, for example.
The operations of Steps S1, S11, and S14 to S18 are substantially common between the first embodiment and the third embodiment. Accordingly, description of operations in Steps S1, S11, S14, and S16 to S18 is to be omitted. Operations in Steps S41 and S15 will be described.

Step S41: Selection step

The treatment liquid L is selected.
The controller 10 selects either the treatment liquid L1 or the treatment liquid L2 based on the coefficient K. The coefficient K is defined by Equation (1).
Here, the coefficient K is set depending on the composition of the treatment liquid L. For example, the coefficient K includes a coefficient KL1 based on the treatment liquid L1 and a coefficient KL2 based on the treatment liquid L2.
Further, the coefficient KL1 and the coefficient KL2 are each set depending on the state of the surface WS of the substrate W. For example, the coefficient KL1 and the coefficient KL2 are each set based on the state of the surface WS of the substrate W in the treatment liquid supply step. For example, the coefficient KL1 and the coefficient KL2 are each set based on the state of the surface WS of the substrate W in the solidified film forming step.
The controller 10 is configured to acquire the coefficient K. The controller 10 may acquire the coefficient K by various methods.
Preferably, the controller 10 designates the treatment liquid L that causes the coefficient K to be equal to or less than the threshold TH. In other words, the controller 10 preferably selects the treatment liquid L satisfying that the coefficient K is equal to or less than the threshold TH. For example, when the coefficient KL1 is equal to or less than the threshold TH, the controller 10 preferably selects the treatment liquid L1. For example, when the coefficient KL2 is equal to or less than the threshold TH, the controller 10 preferably selects the treatment liquid L2.

Step S15: Treatment liquid supply step

The treatment liquid L selected in the selection step is supplied to the substrate W.

When the treatment liquid L1 is selected in the selection step, the treatment liquid L1 is supplied to the substrate W in the treatment liquid supply step. When the treatment liquid L2 is selected in the selection step, the treatment liquid L2 is supplied to the substrate W in the treatment liquid supply step.

When the treatment liquid L1 is selected in the selection step, the treatment liquid L2 is not supplied to the substrate W in the treatment liquid supply step. When the treatment liquid L2 is selected in the selection step, the treatment liquid L1 is not supplied to the substrate W in the treatment liquid supply step.

<3-3. Effect of Third Embodiment>

The substrate treating method is to treat a substrate W on which a pattern P is formed. The substrate treating method includes a selection step, a treatment liquid supply step, a solidified film forming step, and a sublimation step. In the selection step, the treatment liquid L is selected. The treatment liquid L contains a sublimable substance and a solvent. In the treatment liquid supply step, the treatment liquid L selected in the selection step is supplied to the substrate W. In the solidified film forming step, the solvent is evaporated from the treatment liquid L on the substrate W. In the solidified film forming step, the solidified film G is formed on the substrate W. The solidified film G contains the sublimable substance. In the sublimation step, the solidified film G is sublimated. The substrate W is dried by sublimation of the solidified film G.

Here, in the selection step, the treatment liquid L is selected based on the coefficient K. The coefficient K is defined by the interfacial free energies $\gamma LG$, $\gamma GW$, and $\gamma LW$. Accordingly, in the selection step, the treatment liquid L is appropriately selected. Thus, in the solidified film forming step, the solidified film G is suitably formed on the substrate W. Specifically, in the solidified film forming step, the solidified film G smoothly expands on the substrate W. Therefore, in the solidified film forming step, the solvent is efficiently removed from the substrate W. In the solidified film forming step, the treatment liquid L efficiently disappears from the substrate W. Therefore, in the sublimation step, the substrate W is dried in a state where the pattern P formed on the substrate W is suitably protected.

As described above, according to the substrate treating method according to the third embodiment, the substrate W is appropriately treated.

In the selection step, the treatment liquid L that sets the coefficient K to be equal to or less than the threshold TH is selected. Accordingly, in the selection step, the treatment liquid L is more appropriately selected.

The coefficient K is set depending on the state of the surface WS of the substrate W. Accordingly, the coefficient K is appropriately set regardless of the state of the surface WS of the substrate W. Thus, in the selection step, the treatment liquid L is selected depending on the state of the surface WS of the substrate W. In the selection step, the treatment liquid L is appropriately selected regardless of the state of the surface WS of the substrate W. Therefore, in the treatment liquid supply step, the solidified film forming step, and the sublimation step, the substrate W is appropriately treated regardless of the state of the surface WS of the substrate W.

The coefficient K is set based on the state of the surface WS of the substrate W in the treatment liquid supply step. Accordingly, the substrate W is appropriately treated regardless of the state of the surface WS of the substrate W in the treatment liquid supply step.

The coefficient K is set based on the state of the surface WS of the substrate W in the solidified film forming step. Accordingly, the substrate W is appropriately treated regardless of the state of the surface WS of the substrate W in the solidified film forming step.

The coefficient K is set depending on the composition of the treatment liquid L. Accordingly, the coefficient K is appropriately set regardless of the composition of the treatment liquid L. Thus, in the selection step, the treatment liquid L is selected depending on the composition of the treatment liquid L. In the selection step, the treatment liquid L is selected in consideration of the composition of the treatment liquid L. In the selection step, the treatment liquid L is appropriately selected regardless of the composition of the treatment liquid L. Therefore, in the treatment liquid supply step, the solidified film forming step, and the sublimation step, the substrate W is appropriately treated regardless of the composition of the treatment liquid L.

<4. Fourth Embodiment>

A fourth embodiment will be described with reference to the drawings. Like numerals are used to identify like components which are the same as those in the first embodiment, and the components will not particularly be described.

<4-1. Construction and Operation Example of Fourth Embodiment>

The fourth embodiment relates to the treatment liquid evaluating method for evaluating the treatment liquid L. The treatment liquid L is to treat the substrate W on which the pattern P is formed. The treatment liquid L is used in dry treatment of the substrate W on which the pattern P is formed. The treatment liquid L contains a sublimable substance and a solvent. The solvent evaporates from the treatment liquid L, and thus the treatment liquid L becomes the solidified film G containing the sublimable substance.

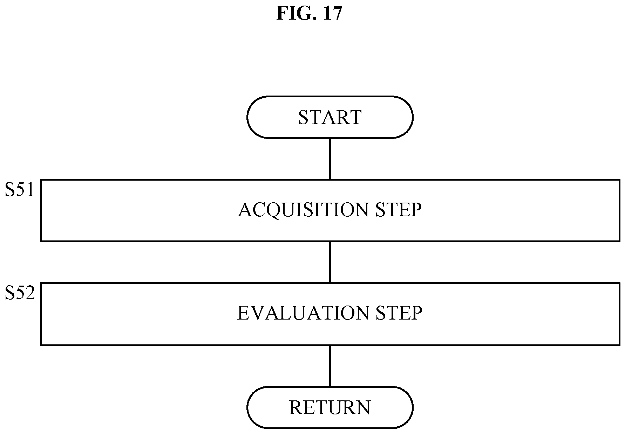

FIG. 17 is a flowchart showing procedures of the treatment liquid evaluating method according to the fourth embodiment. The treatment liquid evaluating method includes an acquisition step and an evaluation step.

S51: Acquisition step

The coefficient K is acquired. The coefficient K is defined by Equation (1).

The coefficient K is acquired by various methods.

For example, the coefficient K is acquired depending on the state of the surface WS of the substrate W.

For example, the coefficient K includes a first coefficient Ka and a second coefficient Kb. For example, in the acquisition step, the first coefficient Ka and the second coefficient Kb are acquired. Here, the first coefficient Ka corresponds to the coefficient K when the surface WS of the substrate W has hydrophobicity. The second coefficient Kb corresponds to the coefficient K when the surface WS of the substrate W has hydrophilicity. The first coefficient Ka and the second coefficient Kb are exemplified in the first embodiment.

For example, the substrate W includes the first substrate Wa and the second substrate Wb. For example, the acquisition step includes a first step, a second step, and a third step. In the first step, the first substrate Wa and the second substrate Wb are prepared. In the second step, the coefficient K is acquired based on the first substrate Wa. For example, in the second step, the coefficient K is calculated based on the first substrate Wa. In the third step, the coefficient K is acquired based on the second substrate Wb. For example, in the third step, the coefficient K is calculated based on the second substrate Wb. Here, the first substrate Wa corresponds to the substrate W having the surface WS that is hydrophobic. The second substrate Wb corresponds to the substrate W having the surface WS that is hydrophilic. The first substrate Wa and the second substrate Wb are exemplified in the first embodiment.

For example, the coefficient K is acquired based on the state of the surface WS of the substrate W and the composition of the treatment liquid L.

For example, the coefficient K is acquired based on the state of the surface WS of the substrate W, the composition of the treatment liquid L, and the composition of the solidified film G. Here, the composition of the solidified film G is estimated from the composition of the treatment liquid L.

For example, the coefficient K is selected from preset information regarding the coefficient K. The preset information regarding the coefficient K is, for example, the table shown in FIG. 12.

S52: Evaluation step

The treatment liquid L is evaluated based on the coefficient K.

For example, the treatment liquid L is evaluated based on the first coefficient Ka. For example, the treatment liquid L is evaluated based on the second coefficient Kb.

For example, the treatment liquid L is classified into a plurality of classes based on the coefficient K. Specifically, the treatment liquid L that causes the coefficient K to be equal to or less than the threshold TH is classified into a first class Q1. The treatment liquid L that causes the coefficient K to be larger than the threshold TH is classified into a second class Q2. In other words, the treatment liquid L satisfying the first condition is classified into the first class Q1. The treatment liquid L not satisfying the first condition is classified into the second class Q2. Here, the first condition is that the coefficient K is equal to or less than the threshold TH.

Reference is made to FIG. 12. The table of FIG. 12 exemplifies the classification of each of the treatment liquids L. The threshold TH is set to 17 mN/m. For example, the coefficient K of Example 1a satisfies the first condition. Accordingly, the treatment liquid L of Example 1a is classified into the first class Q1. For example, the coefficient K of Example 1b does not satisfy the first condition. Accordingly, the treatment liquid L of Example 1b is classified into the second class Q2.

<4-2. Effect of Fourth Embodiment>

The treatment liquid evaluating method is to evaluate the treatment liquid L. The treatment liquid L is to treat the substrate W on which the pattern P is formed. The treatment liquid L contains a sublimable substance and a solvent. When the solvent evaporates from the treatment liquid L, the treatment liquid L becomes the solidified film G. The solidified film G contains the sublimable substance.

The treatment liquid evaluating method includes an acquisition step and an evaluation step. The acquisition step acquires the coefficient K. The coefficient K is defined by the interfacial free energies yLG, yGW, and yLW. In the evaluation step, the treatment liquid L is evaluated based on the coefficient K. Thus, the treatment liquid L is suitably evaluated from the viewpoint of the quality of the treatment of the substrate W.

As described above, according to the treatment liquid evaluating method, the treatment liquid L is appropriately evaluated. The treatment liquid evaluating method is useful in selecting the treatment liquid L.

For example, even when the average collapse rate E is not actually measured, the average collapse rate E is estimated based on the coefficient K. Even when the treatment liquid L is not actually supplied to the substrate W, the quality of the treatment on the substrate W using the treatment liquid L is estimated based on the coefficient K. Thus, the treatment liquid evaluating method is useful in screening the treatment liquid L.

In the acquisition step, the coefficient K is acquired depending on the state of the surface WS of the substrate W. Accordingly, in the acquisition step, the coefficient K is appropriately acquired regardless of the state of the surface WS of the substrate W. Thus, in the evaluation step, the treatment liquid L is evaluated in consideration of the state of the surface WS of the substrate W. In the evaluation step, the treatment liquid L is appropriately evaluated regardless of the state of the surface WS of the substrate W.

The coefficient K includes the first coefficient Ka when the surface WS of the substrate W has hydrophobicity and the second coefficient Kb when the surface WS of the substrate W has hydrophilicity. In the acquisition step, the first coefficient Ka and the second coefficient Kb are acquired. In the evaluation step, the treatment liquid L is evaluated based on the first coefficient Ka and the second coefficient Kb. Specifically, in the evaluation step, the treatment liquid L is evaluated based on the first coefficient Ka. In the evaluation step, the treatment liquid L is evaluated based on the second coefficient Kb. Thus, in the evaluation step, the treatment liquid L is evaluated in consideration of the state of the surface WS of the substrate W. In the evaluation step, the treatment liquid L is appropriately evaluated regardless of the state of the surface WS of the substrate W.

The acquisition step includes a first step, a second step, and a third step. In the first step, the first substrate Wa having the surface WS that is hydrophobic and the second substrate Wb having the surface WS that is hydrophilic are prepared. In the second step, the coefficient K is acquired based on the first substrate Wa. In the third step, the coefficient K is acquired based on the second substrate Wb. Accordingly, in the acquisition step, the coefficient K is appropriately acquired regardless of the state of the surface WS of the substrate W.

In the acquisition step, the coefficient K is acquired based on the state of the surface WS of the substrate W and the composition of the treatment liquid L. Accordingly, in the acquisition step, the coefficient K is appropriately acquired.

In the acquisition step, the coefficient K is acquired based on the state of the surface WS of the substrate W, the composition of the treatment liquid L, and the composition of the solidified film G. Accordingly, in the acquisition step, the coefficient K is more appropriately acquired.

In the acquisition step, the coefficient K is selected from preset information regarding the coefficient K. Accordingly, in the acquisition step, the coefficient K is easily acquired.

In the evaluation step, the treatment liquid L is classified into a plurality of classes based on the coefficient K. For example, in the evaluation step, the treatment liquid L that sets the coefficient K to be equal to or less than the threshold TH is classified into the first class Q1. For example, in the evaluation step, the treatment liquid L that sets the coefficient K to be larger than the threshold TH is classified into the second class Q2. Thus, in the evaluation step, the treatment liquid L is clearly evaluated.

<5. Modified Embodiment>

The present invention is not limited to the first, second, third, and fourth embodiments, and can be modified as follows.

(1) The coefficient K is set based on, for example, the state of the surface WS of the substrate W in the treatment liquid supply step. The coefficient K is set based on, for example, the state of the surface WS of the substrate W in the solidified film forming step. However, the present invention is not limited thereto. The coefficient K may be set based on, for example, the state of the surface WS of the substrate W in at least one of the treatment liquid supply step or the solidified film forming step.

(2) The threshold TH was a constant. However, the present invention is not limited thereto. For example, the threshold TH may be a variable. In the modified embodiment, the first condition can be set more appropriately. Here, the first condition is that the coefficient K is equal to or less than the threshold TH. Thus, the substrate W can be more appropriately treated.

Reference is made to FIG. 1. The projections A each have a width AW and a height AH. A ratio R of the height AH to the width AW is called "aspect ratio R of the projection A" or "aspect ratio R of the pattern P". The threshold TH may be, for example, a variable that depends on the aspect ratio R. In the modified embodiment, the first condition is more appropriately set depending on the aspect ratio R. Thus, the substrate W is more appropriately treated regardless of the aspect ratio R.

For example, the threshold TH may decrease as the aspect ratio R increases. As the aspect ratio R increases, the pattern P collapses more easily. Meanwhile, as the threshold TH decreases, the solidified film G more smoothly expands on the substrate W. Thus, as the threshold TH decreases, the pattern P is more surely protected. Therefore, even when the aspect ratio R is large, the pattern P is suitably protected. Regardless of the aspect ratio R, the substrate W is appropriately treated.

Also in the modified embodiment, the controller 10 may acquire the threshold TH by various methods. The controller 10 reads the threshold TH, for example. The controller 10 selects the threshold TH, for example. The controller 10 calculates the threshold TH, for example. The controller 10 receives the threshold TH, for example.

(3) In the first embodiment, the procedure for acquiring the coefficient K has been exemplified. However, the present invention is not limited thereto. The procedure of acquiring the coefficient K may be changed, as appropriate. For example, in the preparation step, the first substrate Wa may simulate the substrate W in the solidified film forming step. For example, in the preparation step, the second substrate Wb may simulate the substrate W in the solidified film forming step. For example, in the preparation step, the composition of the first standard solution and the composition of the second standard solution may be changed, as appropriate. For example, in the measurement step, a contact angle $\theta 6a$ between the treatment liquid L and the first substrate Wa and a contact angle $\theta 6b$ between the treatment liquid L and the second substrate Wb may be measured. For example, in the calculation step, an equation other than Equations (2) to (5) may be used.

(4) Reference is made to FIG. 14. In the modification step according to the second embodiment, the surface WS of the substrate W is modified to be either hydrophilic or hydrophobic. However, the present invention is not limited thereto.

For example, in the modification step, the surface WS of the substrate W may be modified only to be hydrophobic. When the coefficient Ki is larger than the threshold TH, the controller 10 may determine to perform the hydrophobization step. When the coefficient Ki is larger than the threshold TH, the hydrophobization step may be executed before the treatment liquid supply step. In the modified embodiment, the modification selection step and the hydrophilization step may be omitted. In the modified embodiment, the modification step is simplified.

Alternatively, in the modification step, the surface WS of the substrate W may be modified only to be hydrophilic. Specifically, when the coefficient Ki is larger than the threshold TH, the controller 10 may determine to perform the hydrophilization step. When the coefficient Ki is larger than the threshold TH, the hydrophilization step may be executed before the treatment liquid supply step. In the modified embodiment, the modification selection step and the hydrophobization step may be omitted. In the modified embodiment, the modification step is simplified.

(5) In the substrate treating method according to the second embodiment, the comparison step and the modification selection step are executed after the rotation starting step. However, the present invention is not limited thereto. The timing of executing the comparison step and the modification selection step may be changed, as appropriate. The comparison step and the modification selection step may be executed before the rotation starting step.

(6) In the hydrophobization step according to the second embodiment, hydrofluoric acid was used. However, the present invention is not limited thereto. In the hydrophobization step, instead of hydrofluoric acid, another hydrophobizing agent that modifies the surface of the substrate W to be hydrophobic may be used.

(7) In the hydrophilization step according to the second embodiment, SC1 was used. However, the present invention is not limited thereto. In the hydrophilization step, instead of SC1, another hydrophilizing agent that modifies the surface of the substrate W to be hydrophilic may be used.

(8) Reference is made to FIG. 16. In the selection step according to the third embodiment, for example, when both the coefficient KL1 and the coefficient KL2 are equal to or less than the threshold TH, the controller 10 may select one of the treatment liquids L1 and L2 based on a different criterion. The different criterion is, for example, at least one of the time required for the dry treatment or the cost of the treatment liquid L. The time required for the dry treatment includes, for example, the time required for the treatment liquid supply step, the time required for the solidified film forming step, and the time required for the sublimation step. The cost of the treatment liquid L includes, for example, the cost of the sublimable substance and the cost of the solvent.

(9) Reference is made to FIG. 16. In the substrate treating method according to the third embodiment, the selection step was executed after the rotation starting step and the replacement liquid supply step. However, the present invention is not limited thereto. The timing of executing the selection step may be changed, as appropriate. For example, the selection step may be executed before the replacement liquid supply step. For example, the selection step may be executed before the rotation starting step.

(10) In the solidified film forming step according to the first to third embodiments, the dry gas was not supplied to the substrate W. However, the present invention is not limited thereto. In the solidified film forming step, the dry gas may be supplied to the substrate W. In the solidified film forming step, the dry gas may be supplied to the treatment liquid L on the substrate W.

According to the modified embodiment, in the solidified film forming step, the treatment liquid L on the substrate W is exposed to the dry gas. Accordingly, in the solidified film forming step, the solvent is more efficiently removed from the substrate W. In the solidified film forming step, the treatment liquid L on the substrate W disappears from the substrate W more efficiently. Thus, by the end of the solidified film forming step, the entire treatment liquid L on the substrate W disappears more reliably. Therefore, in the sublimation step, the substrate W is dried in a state where the pattern P is more suitably protected.

(11) The substrate treating method according to the first embodiment includes the chemical liquid supply step, the cleaning liquid supply step, the first rinse liquid supply step, the second rinse liquid supply step, and the replacement liquid supply step. However, the present invention is not limited thereto. For example, at least one of the chemical liquid supply step, the cleaning liquid supply step, the first rinse liquid supply step, the second rinse liquid supply step, or the replacement liquid supply step may be omitted. For example, all of the chemical liquid supply step, the cleaning liquid supply step, the first rinse liquid supply step, the second rinse liquid supply step, and the replacement liquid supply step may be omitted.

The substrate treating method according to the second and third embodiments includes the replacement liquid supply step. However, the present invention is not limited thereto. For example, the replacement liquid supply step may be omitted.

The substrate treating method according to the second embodiment may further include an additional rinse liquid supply step. For example, the additional rinse liquid supply step may be executed after the modification step and before the replacement step. The additional rinse liquid supply step is substantially the same as the first rinse liquid supply step and the second rinse liquid supply step described in the first embodiment, for example.

(12) In the first to third embodiments, a liquid (e.g. a replacement liquid) was present on the substrate W when the treatment liquid supply step was executed. That is, in the treatment liquid supply step, the treatment liquid L was supplied to the substrate W in a non-dried state. However, the present invention is not limited thereto. For example, when the treatment liquid supply step is executed, a liquid (e.g. a replacement liquid) need not be present on the substrate W. For example, in the treatment liquid supply step, the treatment liquid L may be supplied to the substrate W in a dried state.

(13) In the first to third embodiments, the pattern P on the substrate W may be formed on the substrate W, for example, before the substrate W is treated in the treating unit 11. Alternatively, the pattern P may be formed on the substrate W, for example, in the chemical liquid supply step (step S12).

(14) The first to fourth embodiments and modified embodiments described in (1) to (13) above may be further varied as appropriate by replacing or combining their constructions with the constructions of the other modified embodiments.

Reference Signs List

```
1 substrate treating apparatus
10 controller
11 treating unit
13 substrate holder
15a supply unit
15b supply unit
15c supply unit
K coefficient
Ka first coefficient
Kb second coefficient
L treatment liquid
M liquid film
G solidified film
TH threshold
P pattern
W substrate
WS surface of substrate
WS1 upper surface of substrate
A projection
Wa first substrate
Wb second substrate
yLG interfacial free energy at interface between treatment liquid and solidified film
yGW interfacial free energy at interface between solidified film and sub strate
yLW interfacial free energy at interface between treatment liquid and sub strate
yW surface free energy of substrate
```

γWa surface free energy of first substrate
yWb surface free energy of second substrate

**Claims**

1.  A substrate treating method for treating a substrate on which a pattern is formed,
    the substrate treating method comprising:

    a treatment liquid supply step of supplying a treatment liquid containing a sublimable substance and a solvent to the substrate;
    a solidified film forming step of evaporating the solvent from the treatment liquid on the substrate to form a solidified film containing the sublimable substance on the substrate; and
    a sublimation step of sublimating the solidified film,
    wherein a coefficient K defined by the following equation is equal to or less than a threshold:

    $$K = \gamma LG + \gamma GW - \gamma LW$$

    where

    K represents a coefficient,
    yLG represents an interfacial free energy at an interface between the treatment liquid and the solidified film,
    yGW represents an interfacial free energy at an interface between the solidified film and the substrate, and
    γLW represents an interfacial free energy at an interface between the treatment liquid and the substrate.

2.  The substrate treating method according to claim 1, wherein the threshold is a preset constant.

3.  The substrate treating method according to claim 1, wherein the threshold is 17 mN/m.

4.  The substrate treating method according to claim 1, wherein the coefficient K is set depending on a state of a surface of the substrate.

5.  The substrate treating method according to claim 1, wherein each of the interfacial free energy yGW and the interfacial free energy γLW is set depending on a state of a surface of the substrate.

6.  A substrate treating method for treating a substrate on which a pattern is formed,
    the substrate treating method comprising:

    a treatment liquid supply step of supplying a treatment liquid containing a sublimable substance and a solvent to the substrate;
    a solidified film forming step of evaporating the solvent from the treatment liquid on the substrate to form a solidified film containing the sublimable substance on the substrate;
    a sublimation step of sublimating the solidified film; and
    a modification step of modifying a surface of the substrate before the treatment liquid supply step when a coefficient K defined by the following equation is larger than a threshold:

    $$K = \gamma LG + \gamma GW - \gamma LW$$

    where

    K represents a coefficient,
    yLG represents an interfacial free energy at an interface between the treatment liquid and the solidified film,
    yGW represents an interfacial free energy at an interface between the solidified film and the substrate, and
    γLW represents an interfacial free energy at an interface between the treatment liquid and the substrate.

7.  The substrate treating method according to claim 6, wherein the coefficient K is decreased by the modification step.

8. The substrate treating method according to claim 6, wherein, in the modification step, the surface of the substrate is modified to be either hydrophilic or hydrophobic.

9. The substrate treating method according to claim 6, wherein the modification step is not executed when the coefficient K is equal to or less than the threshold.

10. A substrate treating method for treating a substrate on which a pattern is formed,
the substrate treating method comprising:

a selection step of selecting a treatment liquid containing a sublimable substance and a solvent based on a coefficient K defined by the following equation;
a treatment liquid supply step of supplying the treatment liquid selected by the selection step to the substrate;
a solidified film forming step of evaporating the solvent from the treatment liquid on the substrate to form a solidified film containing the sublimable substance on the substrate; and
a sublimation step of sublimating the solidified film:

$$K = \gamma LG + \gamma GW - \gamma LW$$

where

K represents a coefficient,
yLG represents an interfacial free energy at an interface between the treatment liquid and the solidified film,
yGW represents an interfacial free energy at an interface between the solidified film and the substrate, and
$\gamma$LW represents an interfacial free energy at an interface between the treatment liquid and the substrate.

11. The substrate treating method according to claim 10, wherein, in the selection step, the treatment liquid that causes the coefficient K to be equal to or less than a threshold is selected.

12. A treatment liquid evaluating method that evaluates a treatment liquid for treating a substrate on which a pattern is formed,

wherein the treatment liquid contains a sublimable substance and a solvent, and
the solvent evaporates from the treatment liquid, and thus the treatment liquid becomes a solidified film containing the sublimable substance, and
the treatment liquid evaluating method includes:

an acquisition step of acquiring a coefficient K defined by the following equation; and
an evaluation step of evaluating the treatment liquid based on the coefficient K:

$$K = \gamma LG + \gamma GW - \gamma LW$$

where

K represents a coefficient,
yLG represents an interfacial free energy at an interface between the treatment liquid and the solidified film,
yGW represents an interfacial free energy at an interface between the solidified film and the substrate, and
$\gamma$LW represents an interfacial free energy at an interface between the treatment liquid and the substrate.

13. The treatment liquid evaluating method according to claim 12, wherein, in the acquisition step, the coefficient K is acquired depending on a state of a surface of the substrate.

14. The treatment liquid evaluating method according to claim 12, wherein the coefficient K includes a first coefficient when a surface of the substrate has hydrophobicity, and

a second coefficient when a surface of the substrate has hydrophilicity, and

wherein, in the acquisition step, the first coefficient and the second coefficient are acquired, and

in the evaluation step, the treatment liquid is evaluated based on the first coefficient and the second coefficient.

15. The treatment liquid evaluating method according to claim 12, wherein, in the evaluation step, the treatment liquid that causes the coefficient K to be equal to or less than a threshold is classified into a first class, and the treatment liquid that causes the coefficient K to be larger than the threshold is classified into a second class.

**FIG. 1**

**FIG. 2**

EP 4 495 979 A1

FIG. 3

CONTROLLER 10

TRANSPORT MECHANISM 5

TRANSPORT MECHANISM 8

TREATING UNIT 11

ROTATION DRIVING UNIT 14

SUPPLY UNIT 15a, 15b, 15c, 15d, 15e, 15f

VALVE 18a, 18b, 18c, 18d, 18e, 18f

SUPPLY SOURCE 19a

GENERATION UNIT 20

SUPPLY UNIT 22a, 22b

VALVE 24a, 24b

LIQUID FEEDING UNIT 26

PUMP 28

**FIG. 4**

EP 4 495 979 A1

**FIG. 5**

```
                    START

S1  ┌──────────────────────────┐
    │   TREATMENT LIQUID        │
    │   GENERATING STEP         │
    └──────────────────────────┘

            S11  ┌──────────────────────────┐
                 │   ROTATION STARTING STEP  │
                 └──────────────────────────┘

            S12  ┌──────────────────────────┐
                 │   CHEMICAL LIQUID         │
                 │   SUPPLY STEP             │
                 └──────────────────────────┘

            S13  ┌──────────────────────────┐
                 │   FIRST RINSE LIQUID      │
                 │   SUPPLY STEP             │
                 └──────────────────────────┘

            S14  ┌──────────────────────────┐
                 │   REPLACEMENT LIQUID      │
                 │   SUPPLY STEP             │
                 └──────────────────────────┘

            S15  ┌──────────────────────────┐
                 │   TREATMENT LIQUID        │
                 │   SUPPLY STEP             │
                 └──────────────────────────┘

            S16  ┌──────────────────────────┐
                 │   SOLIDIFIED FILM         │
                 │   FORMING STEP            │
                 └──────────────────────────┘

            S17  ┌──────────────────────────┐
                 │   SUBLIMATION STEP        │
                 └──────────────────────────┘

            S18  ┌──────────────────────────┐
                 │   ROTATION STOPPING STEP  │
                 └──────────────────────────┘

                    RETURN
```

**FIG. 6**

**FIG. 7**

**FIG. 8**

**FIG. 9**

FIG. 10

**FIG. 11**

```
                    START

S11 |      ROTATION STARTING STEP

S12 |     CHEMICAL LIQUID SUPPLY STEP

S13 |    FIRST RINSE LIQUID SUPPLY STEP

S21 |     CLEANING LIQUID SUPPLY STEP

S22 |   SECOND RINSE LIQUID SUPPLY STEP

S14 |   REPLACEMENT LIQUID SUPPLY STEP

S15 |    TREATMENT LIQUID SUPPLY STEP

S16 |     SOLIDIFIED FILM FORMING STEP

S17 |          SUBLIMATION STEP

S18 |       ROTATION STOPPING STEP

                    RETURN
```

**FIG. 12**

| EXAMPLE | STATE OF SURFACE WS | SUBLIMABLE SUBSTANCE | SOLVENT | $\gamma$ LW [mN/m] | $\gamma$ GW [mN/m] | $\gamma$ LG [mN/m] | K [mN/m] | E [%] | CLASSIF ICATION |
|---|---|---|---|---|---|---|---|---|---|
| 1a | HYDRO PHOBIC | CYCLOHEXANONE OXIME | ISOPROPYL ALCOHOL | 21.7 | 0.06 | 23.8 | 2.16 | 0.7 | Q1 |
| 1b | HYDRO PHILIC | CYCLOHEXANONE OXIME | ISOPROPYL ALCOHOL | 14.4 | 11.59 | 23.8 | 20.99 | 78.6 | Q2 |
| 2a | HYDRO PHOBIC | CYCLOHEXANONE OXIME | METHANOL | 21.89 | 0.06 | 24.06 | 2.23 | 0.1 | Q1 |
| 2b | HYDRO PHILIC | CYCLOHEXANONE OXIME | METHANOL | 12.79 | 11.59 | 24.06 | 22.86 | 100 | Q2 |
| 3a | HYDRO PHOBIC | CAMPHOR | ISOPROPYL ALCOHOL | 5.32 | 8.41 | 15.1 | 18.19 | 37.4 | Q2 |
| 3b | HYDRO PHILIC | CAMPHOR | ISOPROPYL ALCOHOL | 10.36 | 36.49 | 15.1 | 41.23 | 100 | Q2 |
| 4a | HYDRO PHOBIC | CAMPHOR | METHANOL | 3.89 | 8.41 | 12.83 | 17.35 | 9.95 | Q2 |
| 4b | HYDRO PHILIC | CAMPHOR | METHANOL | 10.36 | 36.49 | 12.83 | 38.96 | 100 | Q2 |

FIG. 13

EP 4 495 979 A1

**FIG. 14**

```
                    ┌──────────────┐
                    │    START     │
                    └──────┬───────┘
                           │
S11  ┌─────────────────────────────────────┐
     │      ROTATION STARTING STEP          │
     └─────────────────┬───────────────────┘
                       │
S31      ╱─────────────────────────╲
        ╱      COMPARISON STEP       ╲──────┐
        ╲                           ╱       │
         ╲─────────────┬───────────╱        │
                       │                    │
             S32      ╱───────────────────╲
                     ╱     MODIFICATION     ╲──────┐
                     ╲    SELECTION STEP    ╱      │
                      ╲─────────┬──────────╱       │
                                │                  │
             S33  ┌───────────────────────┐        │
                  │    HYDROPHOBIZATION    │        │
                  │         STEP           │        │
                  │   (MODIFICATION STEP)  │        │
                  └───────────┬───────────┘        │
                              │        S34  ┌──────────────────────┐
                              │             │   HYDROPHILIZATION    │
                              │             │         STEP          │
                              │             │   (MODIFICATION STEP) │
                              │             └──────────┬───────────┘
                       │      │                        │
                       ◄──────┴────────────────────────┘
S14  ┌─────────────────────────────────────┐
     │       REPLACEMENT LIQUID             │
     │         SUPPLY STEP                  │
     └─────────────────┬───────────────────┘
S15  ┌─────────────────────────────────────┐
     │       TREATMENT LIQUID               │
     │         SUPPLY STEP                  │
     └─────────────────┬───────────────────┘
S16  ┌─────────────────────────────────────┐
     │        SOLIDIFIED FILM               │
     │         FORMING STEP                 │
     └─────────────────┬───────────────────┘
S17  ┌─────────────────────────────────────┐
     │        SUBLIMATION STEP              │
     └─────────────────┬───────────────────┘
S18  ┌─────────────────────────────────────┐
     │     ROTATION STOPPING STEP           │
     └─────────────────┬───────────────────┘
                    ┌──────────────┐
                    │    RETURN     │
                    └──────────────┘
```

**FIG. 15**

EP 4 495 979 A1

# FIG. 16

```
                    ┌─────────────┐
                    │    START    │
                    └─────────────┘
                           │
  S11 ┌─────────────────────────────────────────────┐
      │          ROTATION STARTING STEP              │
      └─────────────────────────────────────────────┘
                           │
  S14 ┌─────────────────────────────────────────────┐
      │        REPLACEMENT LIQUID SUPPLY STEP        │
      └─────────────────────────────────────────────┘
                           │
  S41 ┌─────────────────────────────────────────────┐
      │              SELECTION STEP                  │
      └─────────────────────────────────────────────┘
                           │
  S15 ┌─────────────────────────────────────────────┐
      │        TREATMENT LIQUID SUPPLY STEP          │
      └─────────────────────────────────────────────┘
                           │
  S16 ┌─────────────────────────────────────────────┐
      │        SOLIDIFIED FILM FORMING STEP          │
      └─────────────────────────────────────────────┘
                           │
  S17 ┌─────────────────────────────────────────────┐
      │             SUBLIMATION STEP                 │
      └─────────────────────────────────────────────┘
                           │
  S18 ┌─────────────────────────────────────────────┐
      │          ROTATION STOPPING STEP              │
      └─────────────────────────────────────────────┘
                           │
                    ┌─────────────┐
                    │   RETURN    │
                    └─────────────┘
```

**FIG. 17**

```
              ┌─────────────┐
              │    START    │
              └──────┬──────┘
                     │
S51  ┌───────────────┴───────────────┐
     │       ACQUISITION STEP        │
     └───────────────┬───────────────┘
                     │
S52  ┌───────────────┴───────────────┐
     │       EVALUATION STEP         │
     └───────────────┬───────────────┘
                     │
              ┌──────┴──────┐
              │   RETURN    │
              └─────────────┘
```

## INTERNATIONAL SEARCH REPORT

International application No.

**PCT/JP2023/009827**

| A. | CLASSIFICATION OF SUBJECT MATTER |
|---|---|

***H01L 21/304***(2006.01)i; ***H01L 21/306***(2006.01)i
FI: H01L21/304 651B; H01L21/304 651L; H01L21/306 R; H01L21/304 643A

According to International Patent Classification (IPC) or to both national classification and IPC

| B. | FIELDS SEARCHED |
|---|---|

Minimum documentation searched (classification system followed by classification symbols)

H01L21/304; H01L21/306

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2020-194907 A (SCREEN HOLDINGS CO LTD) 03 December 2020 (2020-12-03) abstract, paragraphs [0103]-[0109], fig. 6 | 1-15 |
| A | JP 2021-9988 A (SCREEN HOLDINGS CO LTD) 28 January 2021 (2021-01-28) | 1-15 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 May 2023** | **30 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/009827**

| Patent document cited in search report | | | Publication date (day/month/year) | Patent family member(s) | | | Publication date (day/month/year) |
|---|---|---|---|---|---|---|---|
| JP | 2020-194907 | A | 03 December 2020 | US | 2022/0238327 | A1 | |
| | | | | abstract, paragraphs [0139]-[0149], fig. 6 | | | |
| | | | | WO | 2020/241022 | A1 | |
| | | | | TW | 202044389 | A | |
| | | | | CN | 113874986 | A | |
| | | | | KR | 10-2022-0014880 | A | |
| JP | 2021-9988 | A | 28 January 2021 | US | 2020/0411309 | A1 | |
| | | | | CN | 112151415 | A | |
| | | | | KR | 10-2389768 | B | |

Form PCT/ISA/210 (patent family annex) (January 2015)

**REFERENCES CITED IN THE DESCRIPTION**

**Patent documents cited in the description**

- JP 2021009988 A